# EUROPÄISCHE PATENTSCHRIFT

(11) **EP 3 956 283 B1**
(45) Veröffentlichungstag und Bekanntmachung des Hinweises auf die Patenterteilung: **20.05.2026**
(21) Anmeldenummer: 19731650.8
(22) Anmeldetag: 12.06.2019
(51) Int. Cl.: C07C 67/03, C07C 69/675, A61P 3/00, A61K 31/22

(54) **VERFAHREN ZUR HERSTELLUNG VON POLYOLBASIERTEN ESTERN VON ACYLVERKAPPTEN 3-HYDROXYCARBONSÄUREN**
PROCESS OF PREPARING POLYOL-BASED ESTERS OF ACYL-CAPPED HYDROXY CARBOXYLIC ACIDS
PROCÉDÉ DE PRÉPARATION D'ESTERS À BASE POLYOL D'ACIDES 3-HYDROXYCARBOXYLIQUES PROTÉGÉS PAR ACYLE

(43) Veröffentlichungstag der Anmeldung: 23.02.2022
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/065278
(87) Internationale Veröffentlichungsnummer: WO 2020/249198

(56) Entgegenhaltungen:
- WO-A1-2010/021766
- WO-A1-2013/150153
- WO-A1-2018/115158
- WO-A1-2018/118369
- WO-A1-2019/063984
- WO-A1-95/09144
- WO-A1-95/09145
- WO-A2-2004/108740
- WO-A2-2006/012490

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Polyolestern bzw. Polyglycerinestern von acylverkappter (acylblockierter) bzw. acetoacetylverkappter 3-Hydroxybuttersäure, sowie die auf diese Weise erhältlichen bzw. hergestellten Reaktionsprodukte (d. h. Polyolester von acylverkappter bzw. acylblockierter 3-Hydroxybuttersäure, nämlich Polyglycerinester von acylverkappter bzw. acylblockierter 3-Hydroxybuttersäure) und deren funktionalisierte (z. B. veresterte) Derivate sowie deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Polyolester von acylverkappter bzw. acylblockierter 3-Hydroxybuttersäure, nämlich Polyglycerinester von acylverkappter bzw. acylblockierter 3-Hydroxybuttersäure) bzw. deren funktionalisierte (z. B. veresterte) Derivate umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*), *Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Polyolester von acylverkappter bzw. acylblockierter 3-Hydroxybuttersäure, nämlich Polyglycerinester von acylverkappter bzw. acylblockierter 3-Hydroxybuttersäure) bzw. deren funktionalisierte (z. B. veresterte) Derivate umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter dem Begriff der Ketokörper fasst man insbesondere die drei Verbindungen Acetoacetat (synonym auch Acetacetat oder 3-Oxobutyrat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure sowie Acetoacetat als physiologischer Vorläufer der 3-Hydroxybuttesäure demgegenüber ein wirksameres pharmazeutisch-pharmakologisches Zielmolekül dar, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure sowie für Acetoacetat (und damit für die physiologisch durch Reduktion von Acetoacetat erhältliche 3-Hydroxybuttersäure oder dessen Salz).

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrücktim Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen sowie zu Acetoacetat (und damit physiologisch zu 3-Hydroxybuttersäure oder deren Salzen) ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2013/150153 A1 betrifft Ketokörper und Ketokörperester zur oralen Verabreichung zu Zwecken der Verbesserung oder zum Erhalt der Muskelkraft, wobei bestimmte Ester von Hydroxybutyratmonomeren organoleptisch akzeptabel sein und zu einer hohen Aufnahme vom Darm ins Blut führen sollen, wodurch ein schneller Anstieg der Hydroxybutyratkonzentration im Blut und eine physiologische Reaktion einschließlich einer verbesserten Leistungsabgabe während des Trainings ermöglicht werden soll, sowie darüber hinaus Zusammensetzungen, welche die Ketokörper oder Ketokörperester enthalten.

Weiterhin betrifft die WO 2004/108740 A2 Verbindungen und Zusammensetzungen, welche (R)-3-Hydroxybutyrat-Derivate enthalten, wobei die Verbindungen und Zusammensetzungen als Nahrungsergänzungsmittel zur Steigerung der körperlichen Leistungsfähigkeit und als Therapeutikum zur Linderung der Symptome von Erkrankungen, insbesondere neurologischen Erkrankungen wie Alzheimer oder ähnlichen Erkrankungen, verwendet werden, sowie deren Herstellungsverfahren, wobei in dem Verfahren beispielsweise ein überkritisches Lösungsmittel wie z. B. überkritisches Kohlendioxid verwendet wird und eine Lipase-katalysierte Veresterungs- oder Umesterungsreaktion durchgeführt wird, um die (R)-3-Hydroxybutyrat-Derivate herzustellen.

Die WO 95/09145 betrifft Zusammensetzungen, welche als Nährstoffe nützlich sein sollen, wobei die Zusammensetzungen vorzugsweise wasserlösliche parenterale Nährstoffe sind und Glycerinester von β-Acyloxybutyraten darstellen, wobei die Zusammensetzungen als Ersatz für Glukose bei der intravenösen Nahrungszufuhr nützlich sein sollen.

Darüber hinaus betrifft die WO 95/09144 A1 Zusammensetzungen, welche als parenterale Nährstoffe nützlich sein sollen, wobei diese Zusammensetzungen wasserlösliche Glycerinester der 3-Hydroxybuttersäure sind, wobei die Zusammensetzungen als Ersatz für Glukose bei der intravenösen Nahrungszufuhr nützlich sein sollen.

Weiterhin betrifft die WO 2018/118369 A1 ein Verfahren zur Behandlung oder Verringerung der Migränesymptome und/oder zur Behandlung und/oder Prophylaxe unter Verwendung von 3-Hydroxybutyatglyceriden.

Die WO 2006/012490 A2 betrifft eine ketogene Verbindung mit der allgemeinen Formel (R(OCH(CH₃)CH₂C(O))ₙO)ₘ-A, wobei n eine ganze Zahl zwischen 1 und 10 bezeichnet, m eine ganze Zahl zwischen 1 und 200.000 bezeichnet, A ein Monosaccharid, Polysaccharid oder Olisaccharid-Rest darstellt und R ausgewählt aus der Gruppe von H, C₁-C₆-Alkyl und Acetoacetyl ist.

Die WO 2010/021766 A1 betrifft eine Verbindung, welche in Bezug auf (3R)-Hydroxybutyl-(3R)-Hydroxybutyratenantiomer angereichertes 3-Hydroxybutyl-3-Hydroxybutyrat der Formel (I) ist, wobei die Verbindungen der Formel (I) ein wirksamer und schmackhafter Vorläufer des Ketokörpers (3R)-Hydroxybutyrat sind und zur Behandlung eines Zustands verwendet werden können, welcher durch einen erhöhten Plasmaspiegel an freien Fettsäuren bei einem Menschen oder Tier verursacht, verschlimmert oder damit verbunden ist (beispielsweise ein Zustand, bei welchem Gewichtsverlust oder Gewichtszunahme eine Rolle spielt) oder aber zur Förderung der Wachsamkeit oder zur Verbesserung der kognitiven Funktion oder zur Behandlung, Verhinderung oder Verringerung der Auswirkungen von Neurodegeneration, Toxizität durch freie Radikale, hypoxische Zustände oder Hyperglykämie eingesetzt werden können.

Die WO 2019/063984 A1 betrifft ein Verfahren zur Unterdrückung von Hunger durch Senkung der Plasma-Ghrelin-Spiegel durch Verabreichung einer Verbindung, welche ausgewählt ist aus: (i) (R)-3-Hydroxybutyrat; (ii) einem Ester von (R)-3-Hydroxybutyrat; und (iii) einem Oligomer von (R)-3-Hydroxybutyrat-Einheiten; oder ein pharmazeutisch verträgliches Salz oder Solvat davon. Die WO 2019/063984 A1 betrifft weiterhin ein Verfahren zur Verabreichung einer solchen Verbindung in einer zur Verringerung des Hungers wirksamen Dosierung, damit ein kosmetisch vorteilhafter Verlust oder eine Aufrechterhaltung des Körpergewichts erfolgt, wobei der Plasma-Ghrelin-Spiegel gesenkt wird.

Schließlich betrifft die WO 2018/115158 A1 eine Verbindung zur Verwendung in einem Verfahren zur Behandlung oder Vorbeugung von Migräne und/oder Symptomen davon, wobei die Verbindung ausgewählt ist aus Beta-Hydroxybuttersäure (βHB) oder einem pharmazeutisch verträglichen Salz davon, Acetoacetat (AcAc) oder einem pharmazeutisch verträglichen Salz davon, einem Stoffwechselvorläufer von βHB oder AcAc, 1,3-Butandiol und einer Verbindung, umfassend eine Acetoacetyl- oder 3-Hydroxybutyrat-Einheit.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Polyolester, nämlich Polyglycerinester, der acylverkappten (= acylblockierten) bzw. acetoavetylverkappten 3-Hydroxybuttersäure sowie deren funktionalisierte Derivate einen effizienten und physiologisch wirksamen bzw. physiologisch kompatiblen Präkursor und/oder Metaboliten für die Ketokörper 3-Hydroxybuttersäure und Acetoacetat bzw. für unter physiologischen Bedingungen reduktiv hieraus erzeugte 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Polyglycerinestern der acetoacetylverkappten 3-Hydroxybuttersäure (beta-Hydroxybuttersäure, 3-Hydroxybutansäure) gemäß Anspruch 1 vor; weitere, insbesondere besondere und/oder vorteilhafte Ausgestaltungen dieses erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - einen Polyglycerinester der acetoacetylverkappten 3-Hydroxybuttersäure (beta-Hydroxybuttersäure, 3-Hydroxybutansäure) gemäß dem diesbezüglichen unabhängigen Anspruch (Anspruch 8) bzw. diesbezügliche Gemische hiervon gemäß dem diesbezüglichen Anspruch (Anspruch 10); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 11); weitere, insbesondere besondere und/oder vorteilhafte Ausgestaltungen dieses Erfindungsaspekts sind Gegenstand des diesbezüglichen Unteranspruchs.

Des Weiteren betrifft die vorliegende Erfindung - gemäß einem **vierten** Aspekt der vorliegenden Erfindung - die Verwendung eines erfindungsgemäßen gegebenenfalls funktionalisierten Polyglycerinesters der acetoacetlyverkappten 3-Hydroxybuttersäure (beta-Hydroxybuttersäure, 3-Hydroxybutansäure) bzw. eines diesbezüglichen Gemischs hiervon zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 13).

Des Weiteren betrifft die vorliegende Erfindung - gemäß einem **fünften** Aspekt der vorliegenden Erfindung - ein Nahrungsmittel und/oder Lebensmittelerzeugnis gemäß dem diesbezüglichen unabhängigen Anspruch (Anspruch 14).

Schließlich betrifft die vorliegende Erfindung - gemäß einem **sechsten** Aspekt der vorliegenden Erfindung - die Verwendung eines erfindungsgemäßen gegebenenfalls funktionalisierten Polyglycerinesters der acetoacetylverkappten 3-Hydroxybuttersäure (beta-Hydroxybuttersäure, 3-Hydroxybutansäure) bzw. eines diesbezüglichen Gemischs hiervon in einem Nahrungsmittel- und/oder Lebensmittelerzeugnis gemäß dem diesbezüglichen unabhängigen Anspruch (Anspruch 15).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Polyglycerinestern von acetoacetylverkappter 3-Hydroxybuttersäure,
wobei mindestens eine Verbindung der allgemeinen Formel (I)

   CH₃ - CH(OR²) - CH₂ - C(O)OR¹ (I)
wobei in der allgemeinen Formel (I) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt und der Rest R² einen Rest CH₃ - C(O) - CH₂ - C(O) - darstellt,
mit mindestens einem Polyglycerin der allgemeinen Formel (IIb)

   HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 4, vorzugsweise 1 oder 2, besonders bevorzugt 1, darstellt,
umgesetzt wird,
wobei die Umsetzung in Abwesenheit von Lösemitteln durchgeführt wird und wobei die Umsetzung in Gegenwart eines Enzyms als Katalysator durchgeführt wird, wobei der Katalysator nach der Umsetzung rezykliert wird,
so dass als Reaktionsprodukt mindestens ein acetoacetylverkappter 3-Hydroxybuttersäure-Polyglycerinester erhalten wird,
wobei bei der Umsetzung gleichzeitig die Verbindung gemäß der allgemeinen Formel (VI)

   R¹ - OH (VI)
gebildet wird, wobei in der allgemeinen Formel (VI) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt; wobei die Verbindung gemäß der allgemeinen Formel (VI) der Umsetzung kontinuierlich entzogen wird.

Nach dem erfindungsgemäßen Verfahren resultiert also ein Polyolester, nämlich ein Polyglycerinester, der in 3-Position (= Hydroxylgruppen-Position) mit einer Acylgruppe bzw. Acetoacetylgruppe verkappten bzw. blockierten 3-Hydroxybuttersäure.

Bei einer Acylgruppe handelt es sich um eine funktionelle Gruppe in der organischen Chemie mit der allgemeinen Struktur A - (C = O) -, wobei der Rest A einen Organyl-Rest (z. B. Alkyl-, Aryl- oder eine heteroaromatische Gruppe etc.) oder ein Wasserstoffatom darstellt. Die Acylgruppe leitet sich formal von Carbonsäuren, Aldehyden und Carbonsäurechloriden ab, in denen eine OH-Gruppe bzw. ein Wasserstoffatom bzw. ein Chlorid durch einen Rest A substituiert ist. Eine Acylierung bezeichnet die Einführung einer solchen Acylgruppe.

Für den Fall, dass (wie im Fall der Erfindung) die Acylierung an einer Hydroxylgruppe (OH-Gruppe) stattfindet (nämlich an der in 3-Position der 3-Hydroxybuttersäure befindlichen OH-Gruppe), wird insgesamt eine Acyloxygruppe gebildet, welche die allgemeine Struktur A - (C = O) - O - hat ("A" wie zuvor definiert).

Erfindungsgemäß ist eine acylverkappte (= acylblockierte) 3-Hydroxybuttersäure also eine in 3-Position (d. h. in Hydroxylgruppen-Position) acylierte Buttersäure bzw. eine in 3-Position acyloxylierte Butansäure. Im Rahmen der vorliegenden Erfindung ist als Acylverkappung speziell eine Acetoacetylverkappung vorgesehen.

Wie zuvor ausgeführt, hat die Anmelderin nämlich vollkommen überraschend herausgefunden, dass die auf diese Weise hergestellten Polyglycerinester von acetoacetylverkappter 3-Hydroxybuttersäure (welche gegebenenfalls zusätzlich auch noch funktionalisiert sein können, wie nachfolgend noch im Detail beschrieben) effiziente, da physiologisch verträgliche Präkursoren und/oder Metabolite der freien 3-Hydroxybuttersäure bzw. deren Salzen oder Ester darstellen, welche pharmazeutisch bzw. klinisch auch in größeren Mengen zum Einsatz kommen können, da sie physiologisch kompatibel sind.

Die vorgenannten, gegebenenfalls funktionalisierten Polyglycerinester von acetoacetylverkappter 3-Hydroxybuttersäure, welche durch das erfindungsgemäße Herstellungsverfahren erstmals in effizienter Weise zugänglich sind, stellen also eine physiologisch und pharmakologisch relevante Alternative zu der freien 3-Hydroxybuttersäure bzw. deren Salzen oder Ester dar (und zudem zu dem weiteren Ketokörper "Acetoacetat").

Die Herstellung derartiger Verbindungen mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich gegebenenfalls funktionalisierte Polyglycerinester von acetoacetylverkappter 3-Hydroxybuttersäure ohne unerwünschte Nebenreaktionen herstellen lassen, insbesondere einstufig.

Das erfindungsgemäße Verfahren ermöglicht somit erstmalig die Bereitstellung untoxischer, gegebenenfalls funktionalisierter Polyglycerinester von acetoacetylverkappter 3-Hydroxybuttersäure aus an sich bekannten, kommerziell verfügbaren und vor allem physiologisch unbedenklichen Komponenten bzw. Edukten (Ausgangsverbindungen). Die resultierenden gegebenenfalls funktionalisierten Polyglycerinester von acetoacetylverkappter 3-Hydroxybuttersäure können physiologisch, insbesondere im Magen und/oder im Darm, aufgespalten werden und das Zielmolekül "3-Hydroxybuttersäure" bzw. deren Salze (und daneben auch den Ketokörper Acetoacetat aus der Acylverkappung, welches physiologisch wiederum weiter zu 3-Hydroxybuttersäure umgesetzt bzw. reduziert werden kann) als Wirkstoff bzw. Wirkkomponente freisetzen bzw. generieren.

Darüber hinaus weisen die vorgenannten, gegebenenfalls funktionalisierten Polyglycerinester von acetoacetylverkappter 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die Polyglycerinester von acetoacetylverkappter 3-Hydroxybuttersäure frei von toxischen Verunreinigungen bereitzustellen.

Bei der physiologischen Spaltung im Magen und/oder Darm wird der gegebenenfalls funktionalisierte Polyglycerinester von acetoacetylverkappter 3-Hydroxybuttersäure in die Ketoverbindungen 3-Hydroxybuttersäure und 3-Oxobutyrat (Acetoacetat bzw. Acetacetat), welches vom Körper weiter zu 3-Hydroxybutyrat reduziert werden kann, gespalten. Durch die Anwesenheit von sowohl 3-Oxobutyratresten als auch 3-Hydroxybutyratresten bzw. 3-Hydroxybuttersäure liegt eine unterschiedlich schnelle Verfügbarkeit bzw. Freisetzung des Wirkstoffs 3-Hydroxybuttersäure vor. Das erfindungsgemäße Reaktionsprodukt weist folglich einen Retard-Effekt auf. Insgesamt weist der erfindungsgemäße gegebenenfalls funktionalisierte Polyglycerinester von acetoacetylverkappter 3-Hydroxybuttersäure somit zwei Ketokörper mit unterschiedlich schnellem Abbau auf. Eine zusätzliche bzw. weiterführende Retardierung liegt zudem dadurch vor, dass die Ketokörper in Form eines Polyolesters vorliegen und somit zur Freisetzung der Wirkstoffe 3-Hydroxybuttersäure in freier Form und Acetoacetat zusätzlich eine Abspaltung vom Polyol erfolgen muss. Insgesamt werden die Wirkstoffe 3-Hydroxybuttersäure und Acetoacetat aus dem erfindungsgemäßen gegebenenfalls funktionalisierten Polyglycerinester von acetoacetylverkappter 3-Hydroxybuttersäure durch einen mehrstufigen Abbau mit Retardierungseffekt freigesetzt.

Darüber hinaus kann bei entsprechender Auswahl der Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern bzw. zu erhalten, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern bzw. zu erhalten.

Das erfindungsgemäße Herstellungsverfahren führt üblicherweise zu einem Gemisch verschiedener Polyglycerinester von acetoacetylverkappter 3-Hydroxybuttersäure, d. h. zu einem Gemisch von mindestens zwei, insbesondere mindestens drei voneinander verschiedenen Polyglycerinestern der acetoacetylverkappten 3-Hydroxybuttersäure. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Polyolester, d. h. Mono-, Di-, Tri- usw. Polyolester der acetoacetylverkappten 3-Hydroxybuttersäure, oder aber Fraktionierung in Fraktionen mit angereichertem und abgereichertem Anteil einzelner etc.).

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren einfach zugängliche Edukte und ermöglicht darüber hinaus eine relativ einfache Verfahrensführung auch bei großtechnischer Umsetzung. Dennoch werden im Rahmen des erfindungsgemäßen Herstellungsverfahrens exzellente Ausbeuten erzielt, wobei in gleicher Weise die Bildung von Nebenprodukten minimiert (keine signifikanten Mengen an Nebenprodukten) bzw. vermieden wird. Die eingesetzten Edukte sind weiterhin selbst physiologisch kompatibel und sogar pharmazeutisch wirksam, sodass gegebenenfalls noch vorhandene Edukte im Reaktionsprodukt verbleiben können und keine bzw. kaum Aufreinigungsverfahrensschritte notwendig sind. Grundsätzlich ist es aber möglich und kann unter bestimmten Voraussetzungen, insbesondere im Hinblick auf die organoleptischen Eigenschaften, zweckdienlich sein, die Edukte aus dem Reaktionsprodukt zu entfernen.

Im Gegensatz zu herkömmlichen Herstellungsverfahren des Standes der Technik kommt also das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Das erfindungsgemäße Verfahren wird in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Im Rahmen der vorliegenden Erfindung ist es bevorzugt, wenn die Verbindung der allgemeinen Formel (I) in racemischer Form oder Form des (R)-Enantiomers eingesetzt wird. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) 3-Acetylacetobuttersäureethylester (Ethyl-3-acetylaceto-butyrat) der Formel CH₃- CH(OR²) - CH₂ - C(O)OC₂H₅, wobei der Rest R² die zuvor angegebene Bedeutung hat, eingesetzt wird. Dies ermöglicht eine besonders effiziente Verfahrensführung und hohe Ausbeuten mit minimierter bzw. unterdrückter Nebenproduktbildung.

Bei dem erfindungsgemäßen Verfahren wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird die Umsetzung in Gegenwart eines Enzyms als Katalysator durchgeführt. Dabei wird der Katalysator nach der Umsetzung rezykliert.

Wie zuvor ausgeführt, wird im Rahmen des erfindungsgemäßen Herstellungsverfahrens die Umsetzung in Gegenwart eines Enzyms als Katalysator durchgeführt.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich das als Katalysator eingesetzte Enzym insbesondere ableiten von *Candida antarctica, Mucor miehei* (*Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann das Enzym in immobilisierter Form, immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor dargelegt, ist es vorgesehendas Enzym nach der Umsetzung zu rezyklieren.

Erfindungsgemäß wird die Umsetzung im Rahmen des erfindungsgemäßen Herstellungsverfahrens in Gegenwart eines Enzyms als Katalysator durchgeführt; dabei ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

IDie Menge des eingesetzten Enzyms kann in weiten Bereichen variieren. Insbesondere kann das Enzym in Mengen, bezogen auf die Gesamtmenge der Ausgangsverbindungen (I) und (IIb), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Auch der angewendete Druckbereich kann in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I), bezogen auf die Hydroxylgruppen des Polyglycerins (IIb), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I) und das Polyglycerin (IIb) in einem Molverhältnis von Verbindung der allgemeinen Formel (I) / Polyglycerin (IIb) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Was die im Rahmen des erfindungsgemäßen Verfahrens einsetzbare Verbindung der allgemeinen Formel (I) angelangt so ist es insbesondere bevorzugt, wenn die als Ausgangsverbindung eingesetzte Verbindung der allgemeinen Formel (I) eine acetoacetylverkappte 3-Hydroxybuttersäure oder deren Salz oder Ester ist.

Gemäß einer besonderen Ausführungsform der vorliegenden Erfindung kann es insbesondere vorgesehen sein, dass die als Ausgangsverbindung eingesetzte Verbindung der allgemeinen Formel (I), insbesondere die acetoacetylverkappte 3-Hydroxybuttersäure, erhalten wird bzw. erhältlich ist durch die Umsetzung einer Verbindung der allgemeinen Formel (IV)

CH₃ - CH(OH) - CH₂ - C(O)OR¹ (IV)

wobei in der allgemeinen Formel (IV) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt,
mit mindestens einer Verbindung der allgemeinen Formel (V)

   CH₃ - C(O) - CH₂ - C(O)OR³ (V)
wobei in der allgemeinen Formel (V) der Rest R³ ein C₁-C₄-Alkyl, insbesondere Methyl oder Ethyl, bevorzugt Ethyl, darstellt.

Insbesondere kann die Umsetzung der Verbindung der allgemeinen Formel (IV), wie zuvor definiert, mit der Verbindung der allgemeinen Formel (V), wie zuvor definiert, in Abwesenheit von Lösemitteln und/oder ohne jedwedes Lösemittel durchgeführt werden. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer besonderen Ausführungsform der vorliegenden Erfindung kann die Umsetzung der Verbindung der allgemeinen Formel (IV), wie zuvor definiert, mit der Verbindung der allgemeinen Formel (V), wie zuvor definiert, in Gegenwart eines Katalysators, insbesondere eines Enzyms und/oder eines metallhaltigen und/oder metallbasierten, sauren oder basischen Katalysators, vorzugsweise in Gegenwart eines Enzyms, durchgeführt werden. Dies hat den Vorteil, dass insbesondere die Bildung von Nebenprodukten unterdrückt bzw. verringert wird und weiterhin die Umsetzungsgeschwindigkeit erhöht wird. Bei dieser Ausführungsform ist es bevorzugt, wenn der Katalysators nach der Umsetzung rezykliert wird.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (V) und die Verbindung der allgemeinen Formel (IV) in einem Molverhältnis von Verbindung der allgemeinen Formel (V) / Verbindung der allgemeinen Formel (IV) in einem Bereich von 1,1 : 1 bis 10 : 1, bevorzugt in einem Bereich von 1,5 : 1 bis 9 : 1, insbesondere in einem Bereich von 2 : 1 bis 8 : 1, vorzugsweise in einem Bereich von 3 : 1 bis 6 : 1, eingesetzt werden. Auf diese Weise wird einer Nebenproduktbildung, insbesondere der Bildung von dimerer 3-Hydroxybuttersäure und deren acylverkappten Derivaten, in effizienter Weise entgegengewirkt.

Gemäß dem erfindungsgemäßen Verfahren wird ein Polyglycerin der allgemeinen Formel (IIb)

HO - CH₂ - CH(OH) - CH₂ - [O - CH₂ - CH(OH) - CH₂]ₚ - OH (IIb)

eingesetzt, wobei in der allgemeinen Formel (IIb) die Variable p eine ganze Zahl von 1 bis 4, vorzugsweise 1 oder 2, besonders bevorzugt 1, darstellt.

Gemäß einer weiteren besonderen Ausführungsform des erfindungsgemäßen Verfahrens kann das Polyglycerin (IIb) ein Diglycerin der Formel (IIc)

HO - CH₂ - CH(OH) - CH₂ - O - CH₂ - CH(OH) - CH₂ - OH (IIc)

sein.

Erfindungsgemäß ist das Polyglycerin (IIb) kein Propan-1,2,3-triol, d. h., das Polyglycerin (IIb) ist kein Glycerin.

Gemäß einer bevorzugten Ausführungsform der vorliegenden Erfindung betrifft die vorliegende Erfindung gemäß diesem Erfindungsaspekt ein Verfahren zur Herstellung von Polyglycerinestern von acetoacetlyverkappter 3-Hydroxybuttersäure (beta-Hydroxybuttersäure, 3-Hydroxybutansäure), insbesondere wie zuvor definiert,
wobei mindestens eine Verbindung der allgemeinen Formel (I)

   CH₃- CH(OR²) - CH₂ - C(O)OR¹ (I)
wobei in der allgemeinen Formel (I) der Rest R¹ Ethyl darstellt und der Rest R² einen Rest CH₃ - C(O) - CH₂ - C(O) - darstellt,
mit mindestens einem Polyglycerin der allgemeinen Formel (IIb)

   HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 4, vorzugsweise 1 oder 2, besonders bevorzugt 1, darstellt,
so dass als Reaktionsprodukt ein oder mehrere acetoacetylverkappte 3-Hydroxybuttersäure-Polyglycerinester erhalten werden.

Eine erfindungsgemäß besonders bevorzugte Vorgehensweise wird durch das nachfolgende Reaktions- bzw. Syntheseschema (mit "EtOH" = Ethanol und "Kat" = Katalysator) veranschaulicht (wobei in Abhängigkeit von der Reaktionsführung entweder einzelne der Ester oder ein Gemisch von zwei oder mehreren hiervon erhalten werden):

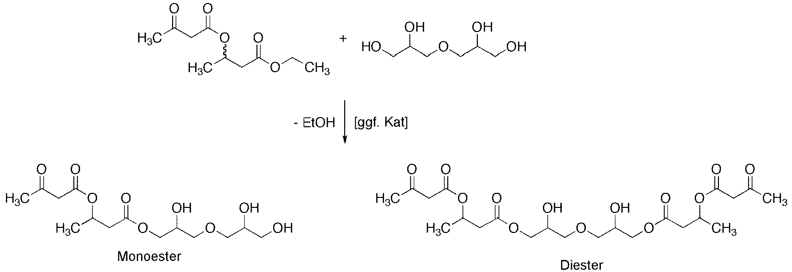

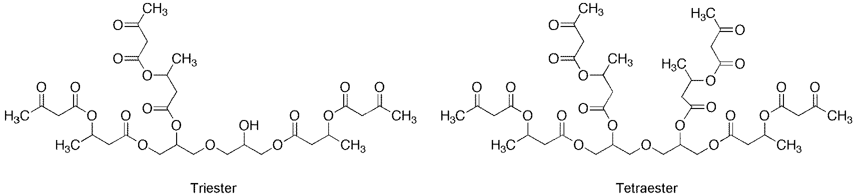

Im Rahmen des erfindungsgemäßen Verfahrens wird bei der Umsetzung gleichzeitig die Verbindung gemäß der allgemeinen Formel (VI)

R¹ - OH (VI)

gebildet, wobei in der allgemeinen Formel (VI) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt.

Insbesondere ist es in diesem Zusammenhang vorgesehen, dass die Verbindung gemäß der allgemeinen Formel (VI) der Umsetzung kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Im Rahmen des erfindungsgemäßen Herstellungsverfahrens kann das Reaktionsprodukt, insbesondere die Zusammensetzung des Reaktionsprodukts, insbesondere das Vorhandensein mehrerer und/oder verschiedener acetoacetylverkappter 3-Hydroxybuttersäure-Polyglycerinester und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Vorsehen eines Katalysators und dessen Auswahl in Bezug auf Art und/oder Menge und/oder Auswahl der Mengen der Ausgangsverbindungen (Edukte) und/oder Vorsehen der Entfernung der Verbindung gemäß der allgemeinen Formel (VI), wie zuvor definiert.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (II) aus dem Reaktionsprodukt abgetrennt und anschließend rezykliert werden. Hierdurch wird eine besonders ökonomische Verfahrensführung ermöglicht.

Gemäß einer besonders bevorzugten Ausführungsform der vorliegenden Erfindung betrifft die vorliegende Erfindung gemäß diesem Erfindungsaspekt ein Verfahren zur Herstellung von Polyglycerinestern von acetoacetylverkappter 3-Hydroxybuttersäure, insbesondere ein Verfahren wie zuvor definiert,
(a) wobei in einem ersten Verfahrensschritt (a) mindestens eine Verbindung der allgemeinen Formel (IV)

   CH₃ - CH(OH) - CH₂ - C(O)OR¹ (IV)

   wobei in der allgemeinen Formel (IV) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt,
   mit mindestens einer Verbindung der allgemeinen Formel (V)

      CH₃ - C(O) - CH₂ - C(O)OR³ (V)
   wobei in der allgemeinen Formel (V) der Rest R³ ein C₁-C₄-Alkyl, insbesondere Methyl oder Ethyl, bevorzugt Ethyl, darstellt, umgesetzt wird, sodass eine Verbindung der allgemeinen Formel (I)

      CH₃ - CH(OR²) - CH₂ - C(O)OR¹ (I)
   wobei in der allgemeinen Formel (I) der Rest R¹ die zuvor angegebene Bedeutung hat und der Rest R² einen Rest CH₃- C(O) - CH₂ - C(O) - darstellt, gebildet wird; und nachfolgend
(b) in einem zweiten Verfahrensschritt (b) die auf diese Weise erhaltene Verbindung der allgemeinen Formel (I), wie zuvor definiert, mit mindestens einem Polyglycerin (IIb), wie zuvor definiert, umgesetzt wird,
   sodass als Reaktionsprodukt mindestens ein acetoacetylverkappter 3-Hydroxybuttersäure-Polyglycerinester erhalten wird.

Eine erfindungsgemäß besonders bevorzugte Vorgehensweise, bestehen aus der Synthese des acetoacetylverkappten 3-Hydroxybuttersäureethylesters und der anschließenden Umsetzung mit Diglycerin, wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden):

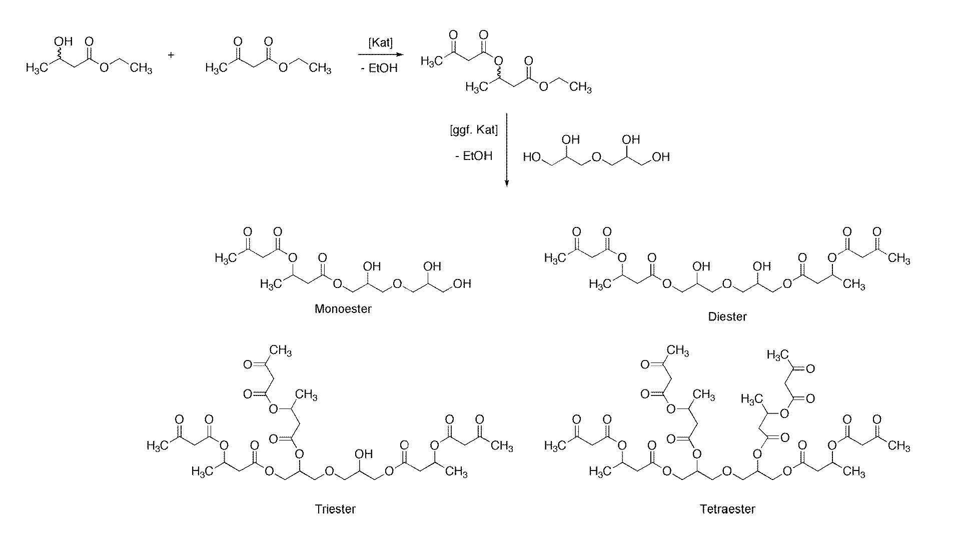

Gemäß einer besonderen Ausführungsfrom des erfindungsgemäßen Herstellungsverfahrens kann insbesondere derart vorgegangen werden, dass im Reaktionsprodukt nach erfolgter Umsetzung noch vorhandene Hydroxylgruppen zumindest teilweise, vorzugsweise vollständig, funktionalisiert, insbesondere verestert, werden.

Mit anderen Worten kann sich gemäß einer besonderen erfindungsgemäßen Ausführungsfrom der Umsetzung eine teilweise, insbesondere vollständige Funktionalisierung, insbesondere Veresterung, noch vorhandener hydroxylgruppen anschließen.

Bei dieser besonderen Ausführungsfrom des erfindungsgemäßen Verfahrens kann insbesondere die Funktionalisierung, insbesondere Veresterung, der Hydroxyl- gruppen im Reaktionsprodukt mit mindestens einem Carbonsäureanhydrid der allgemeinen Formel (VII)

R⁴-O-R⁴ (VII)

wobei in der allgemeinen Formel (VII) der Rest R⁴ jeweils unabhängig voneinander, gleich oder verschieden, einen Rest vom Typ lineares (geradkettiges) oder verzweigtes, gesättigtes oder ein- oder mehrfach ungesättigtes (C₁-C₃₃-Alkyl)- C(O)-, insbesondere (C₄-C₃₃-Alkyl)-C(O)-, vorzugsweise (C₇-C₃₃-Alkyl)- C(O)-, darstellt, durchgeführt werden.

Bei dieser besonderen Ausführungsform des erfindungsgemäßen Verfahrens ist es bevorzugt, wenn in der allgemeinen Formel (VII) der Rest R⁴ jeweils unabhängig voneinander, gleich oder verschieden, ein Fettsäurerest, insbesondere ein C₅-C₃₄-Fettsäurerest, vorzugsweise ein C₈-C₃₄-Fettsäurerest, ist.

Weiterhin ist es bei dieser besonderen Ausführungsform des erfindungsgemäßen Verfahrens bevorzugt, wenn das Carbonsäureanhydrid der allgemeinen Formel (VII) ein Fettsäureanhydrid, insbesondere ein C₅-C₃₄-Fettsäureanhydrid, vorzugsweise ein C₈-C₃₄-Fettsäureanhydrid, ist.

Insbesondere ist es bei dabei bevorzugt, wenn als Carbonsäureanhydrid der allgemeinen Formel (VII) eine Verbindung eingesetzt wird, bei welcher die Reste R⁴ identisch sind. Mit anderen Worten wird als Carbonsäureanhydrid der allgemeinen Formel (VII) ein symmetrisches Carbonsäureanhydrid eingesetzt.

Gemäß einer alternativen Ausführungsform ist es bevorzugt, wenn als Carbonsäureanhydrid der allgemeinen Formel (VII) eine Verbindung eingesetzt wird, bei welcher die Reste R⁴ voneinander verschieden sind. Mit anderen Worten wird als Carbonsäureanhydrid der allgemeinen Formel (VII) ein unsymmetrisches Carbonsäureanhydrid eingesetzt.

Erfindungsgemäß kann die Funktionalisierung der nach erfolgter Umsetzung noch vorhandenen Hydroxylgruppen im Reaktionsprodukt mit dem mindestens einen Carbonsäureanhydrid der allgemeinen Formel (VII) bei Temperaturen im Bereich von 60 bis 150 °C, insbesondere im Bereich von 70 bis 120 °C, vorzugsweise im Bereich von 80 bis 100 °C, durchgeführt werden.

Die erfindungsgemäße Funktionalisierung der nach erfolgter Umsetzung noch vorhandenen Hydroxylgruppen im Reaktionsprodukt mit dem mindestens einen Carbonsäureanhydrid der allgemeinen Formel (VII) kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Insbesondere ist es bevorzugt, wenn die Funktionalisierung der nach erfolgter Umsetzung noch vorhandenen Hydroxylgruppen im Reaktionsprodukt mit dem mindestens einen Carbonsäureanhydrid der allgemeinen Formel (VII) in Abwesenheit von Lösemitteln und/oder ohne jedwedes Lösemittel durchgeführt wird. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Im Rahmen der erfindungsgemäßen Funktionalisierung der nach erfolgter Umsetzung noch vorhandenen Hydroxylgruppen im erfindungsgemäßen Reaktionsprodukt mit dem mindestens einen Carbonsäureanhydrid der allgemeinen Formel (VII) wird gleichzeitig eine Verbindung gemäß der allgemeinen Formel (VIII)

R⁴ - OH (VIII)

wobei der Rest R⁴ die zuvor angegebene Bedeutung hat, gebildet.

Insbesondere ist es in diesem Zusammenhang bevorzugt, wenn die Verbindung gemäß der allgemeinen Formel (VIII) während oder nach erfolgter Umsetzung, insbesondere nach erfolgter Umsetzung, entfernt wird, vorzugsweise destillativ.

Im Anschluss an die erfindungsgemäßen Funktionalisierung der nach erfolgter Umsetzung noch vorhandene Hydroxylgruppen im Reaktionsprodukt mit dem mindestens einen Carbonsäureanhydrid der allgemeinen Formel (VII) kann das erhaltene Produkt weiteren üblichen bzw. an sich bekannten Aufreinigungs- bzw. Aufarbeitungsschritten unterzogen werden.

In diesem Zusammenhang schließt sich der Funktionalisierung eine Destillation und/oder eine Chromatographie, vorzugsweise eine Destillation, an. Insbesondere werden dabei gegebenenfalls noch vorhandene Edukte und Reaktionsnebenprodukte, insbesondere Verbindungen gemäß der allgemeinen Formel (VIII), abdestilliert.

Im Rahmen der erfindungsgemäßen Funktionalisierung ist es vorgesehen, dass für den Fall, dass in der allgemeinen Formel (VII) die Reste R⁴ voneinander verschieden sind, und/oder für den Fall, dass in der allgemeinen Formel (VII) die Reste R⁴ jeweils einen Alkylrest mit mehr als zwei Kohlenstoffatomen darstellen, das Carbonsäureanhydrid der allgemeinen Formel (VII) erhältlich ist und/oder erhalten wird durch die Umsetzung von Essigsäureanhydrid (Acetanhydrid) mit mindestens einer Carbonsäure, insbesondere Fettsäure, der allgemeinen Formel (VIII)

R⁴ - OH (VIII)

wobei der Rest R⁴ die zuvor angegebene Bedeutung hat.

In diesem Zusammenhang erfolgt die Umsetzung von Essigsäureanhydrid (Acetanhydrid) mit der mindestens einen Carbonsäure, insbesondere Fettsäure, der allgemeinen Formel (VIII) entsprechend der Reaktionsgleichung 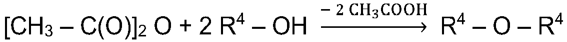 wobei der Rest R⁴ die zuvor angegebene Bedeutung hat, jedoch mit der Maßgabe, dass die Reste R⁴ voneinander verschieden sind, und/oder dass die Reste R⁴ jeweils unabhängig voneinander, einen Alkylrest mit mehr als zwei Kohlenstoffatomen darstellen.

Gemäß einer besonderen Ausführungsform dieses erfindungsgemäßen Verfahrens wird ein symmetrisches Carbonsäureanhydrid der allgemeinen Formel (VII) hergestellt. Mit anderen Worten sind in der allgemeinen Formel (VII) die Reste R⁴ identisch und stellen einen Alkylrest mit mehr als zwei Kohlenstoffatomen dar. Gemäß einer alternativen besonderen Ausführungsform dieses erfindungsgemäßen Verfahrens wird ein unsymmetrisches Carbonsäureanhydrid der allgemeinen Formel (VII) hergestellt. Somit sind in der allgemeinen Formel (VII) die Reste R⁴ voneinander verschieden, vorzugsweise stellen in der allgemeinen Formel (VII) die Reste R⁴ jeweils einen Alkylrest mit mehr als zwei Kohlenstoffatomen dar.

Eine erfindungsgemäß besonders bevorzugte Vorgehensweise, welche eine Funktionalisierung, insbesondere Veresterung, noch vorhandener Hydroxylgruppen (vollständig oder partiell) im Anschluss an die Umsetzung vorsieht, wird durch das nachfolgende Reaktions- bzw. Syntheseschema veranschaulicht (wobei in Abhängigkeit von der Reaktionsführung bei der Umsetzung entweder einzelne Ester oder ein Gemisch von zwei oder mehreren hiervon erhalten werden und wobei in dem nachfolgenden Reaktions- bzw. Syntheseschema der Rest R Wasserstoff oder einen Rest der Formel CH₃ - (CH₂)_{x = 0-28} - C(O) - bezeichnet):

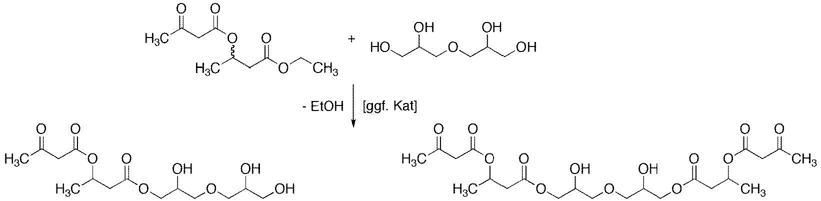

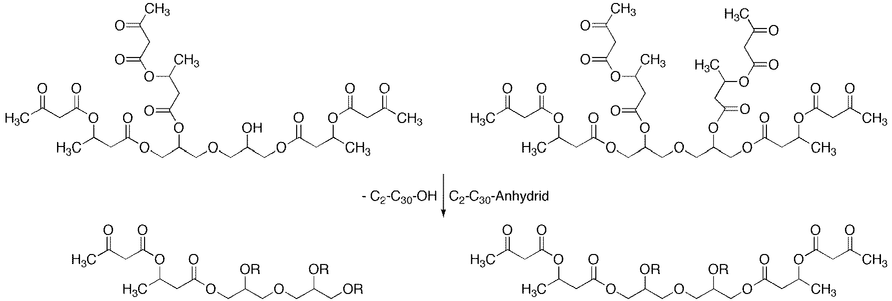

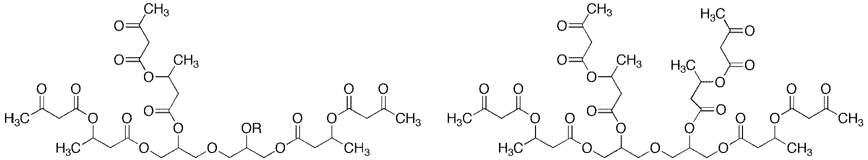

Gemäß einer alternativen Ausführungsform der Funktionalisierung, insbesondere Veresterung, der nach erfolgter Umsetzung noch vorhandenen Hydroxylgruppen im Reaktionsprodukt kann diese insbesondere durch Reaktion mit mindestens einer Carbonsäure und/oder einem Carbonsäureester der allgemeinen Formel (IX)

R⁴ - O - R⁸ (IX)

wobei in der allgemeinen Formel (IX)
- der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt,
- der Rest R⁸ Wasserstoff oder ein C₁-C₄-Alkyl, insbesondere Methyl oder Ethyl, bevorzugt Wasserstoff, darstellt,
durchgeführt werden.

In diesem Zusammenhang ist es insbesondere bevorzugt, wenn die Carbonsäure und/oder der Carbonsäureester der allgemeinen Formel (IX) eine Fettsäure und/oder einen Fettsäureester, insbesondere eine C₅-C₃₄-Fettsäure und/oder einen C₅-C₃₄-Fettsäureester, vorzugsweise eine C₈-C₃₄-Fettsäure und/oder einen C₈-C₃₄-Fettsäureester, darstellt.

Insbesondere wird diese erfindungsgemäße Ausführungsform der Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer bevorzugten Ausführungsform wird die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) in Gegenwart eines Katalysators, insbesondere eines Enzyms und/oder eines metallhaltigen und/oder metallbasierten, sauren oder basischen Katalysators, durchgeführt.

In diesem Zusammenhang ist es insbesondere bevorzugt, wenn der Katalysator nach der Umsetzung rezykliert wird.

Wie zuvor ausgeführt, kann gemäß einer bevorzugten Ausführungsform die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) in Gegenwart eines Enzyms als Katalysator durchgeführt werden.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen dieser besonderen Ausführungsform kann sich das als Katalysator eingesetzte Enzym ableitet von *Candida antarctica, Mucor miehei* (*Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Dabei ist es insbesondere bevorzugt, wenn das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird.

Wie bereits im Zusammenhang mit dem Katalysator im Allgemeinen dargelegt, ist es bevorzugt, wenn das Enzym nach der Umsetzung rezykliert wird.

Sofern die erfindungsgemäße Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (IX) in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist. In diesem Zusammenhang sind die Ausgangsverbindungen der acetoacetylverkappte 3-Hydroxybuttersäure-Polyglycerinester und die Carbonsäure und/oder der Carbonsäureester der allgemeinen Formel (IX).

Wenn gemäß einer besonderen Ausführungsform der vorliegenden Erfindung die Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (IX) in Gegenwart eines Enzyms als Katalysator durchgeführt wird, kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der erfindungsgemäßen Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (IX) kann die Funktionalisierung in Gegenwart eines metallhaltigen und/oder metallbasierten, sauren oder basischen Katalysators durchgeführt werden.

Gemäß dieser alternativen Ausführungsform der erfindungsgemäßen Funktionalisierung, wonach die Funktionalisierung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es auch bei dieser Ausführungsform der Funktionalisierung bevorzugt, wenn der Katalysator auf Basis des metallhaltigen und/oder metallbasierten sauren oder basischen Katalysators nach der Umsetzung rezykliert wird.

Wenn gemäß dieser besonderen Ausführungsform der vorliegenden Erfindung die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator auf Basis eines metallhaltigen und/oder metallbasierten, sauren oder basischen Katalysators in Mengen, bezogen auf die Gesamtmenge der Ausgangsverbindungen, im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist. In diesem Zusammenhang sind die Ausgangsverbindungen der acetoacetylverkappte 3-Hydroxybuttersäure-Polyolester und die Carbonsäure und/oder der Carbonsäureester der allgemeinen Formel (IX).

Wenn gemäß dieser besonderen Ausführungsform der vorliegenden Erfindung die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) in Gegenwart eines metallhaltigen und/oder metallbasierten, sauren oder basischen Katalysators erfolgt, kann der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Im Rahmen dieser besonderen Ausführungsform der Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (IX) wird gleichzeitig eine Verbindung der allgemeinen Formel (XI)

R⁸ - OH (XI)

wobei der Rest R⁸ die zuvor angegebene Bedeutung hat, gebildet.

Insbesondere ist es in diesem Zusammenhang bevorzugt, wenn die Verbindung der allgemeinen Formel (XI) während oder nach erfolgter Umsetzung, insbesondere während der Umsetzung, entfernt wird, vorzugsweise destillativ. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Gemäß einer besonderen Ausführungsform des erfindungsgemäßen Verfahrens können die in dem Reaktionsprodukt durch das zuvor beschriebene Verfahren eingeführten Estergruppen einer teilweisen Umesterung mittels einer Verbindung der allgemeinen Formel (IX), wie zuvor definiert, unterzogen werden. Mit anderen Worten können die in dem Reaktionsprodukt durch das zuvor beschriebene Verfahren eingeführten Estergruppen teilweise durch einen Rest R⁴ mit der zuvor angegebenen Bedeutung mittels Umesterung ausgetauscht werden.

In diesem Zusammenhang kann die Umesterung gemäß dieser besonderen Ausführungsform der vorliegenden Erfindung unter Reaktionsbedingungen, wie zuvor für die erfindungsgemäße Funktionalisierung der nach erfolgter Umsetzung noch vorhandenen Hydroxylgruppen im Reaktionsprodukt mit mindestens einer Carbonsäure und/oder einem Carbonsäureester, erfolgen.

Im Rahmen der vorliegenden Erfindung ist auch eine Funktionalisierung, insbesondere Fettsäurefunktionalierung, der Polyglycerinester von acetoacetylverkappter 3-Hydroxybuttersäure vorgesehen. Zu diesem Zweck kann im Rahmen der Erfindung ein Verfahren zur Herstellung von funktionalisierten, insbesondere fettsäurefunktionalisierten, vorzugsweise C₅-C₃₄-fettsäurefunktionalisierten, bevorzugt C₈-C₃₄-fettsäurefunktionalisierten, Polyglycerinestern von acetoacetylverkappter 3-Hydroxybuttersäure (beta-Hydroxybuttersäure, 3-Hydroxybutansäure) zum Einsatz kommen,
(A) wobei gemäß einer (ersten) Syntheseroute (A) zunächst in einem ersten Verfahrensschritt mindestens eine Verbindung der allgemeinen Formel (I)

   CH₃- CH(OR²) - CH₂ - C(O)OR¹ (I)

   wobei in der allgemeinen Formel (I) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt und der Rest R² einen Rest CH₃ - C(O) - CH₂ - C(O) - darstellt,
   mit mindestens einem Polyglycerin, insbesondere wie zuvor definiert, umgesetzt wird,
   gefolgt von einem zweiten Verfahrensschritt, wobei der zweite Verfahrensschritt

   (i) eine zumindest teilweise Funktionalisierung, insbesondere eine zumindest teilweise Veresterung, noch vorhandener Hydroxylgruppen mittels mindestens einer Fettsäure und/oder deren Ester oder Anhydrid, insbesondere mittels mindestens einer C₅-C₃₄-Fettsäure und/oder deren Ester oder Anhydrid, vorzugsweise mittels mindestens einer C₈-C₃₄-Fettsäure und/oder deren Ester oder Anhydrid, und/oder
   (ii) eine teilweise Umesterung von im ersten Verfahrensschritt eingeführten Estergruppen mittels mindestens einer Fettsäure und/oder deren Ester, insbesondere mittels mindestens einer C₅-C₃₄-Fettsäure und/oder deren Ester, vorzugsweise mittels mindestens einer C₈-C₃₄-Fettsäure und/oder deren Ester
   umfasst;
   oder aber
(B) wobei gemäß einer (zweiten, zu (A) alternativen) Syntheseroute (B) zunächst in einem ersten Verfahrensschritt mindestens ein Polyglycerin, insbesondere wie zuvor definiert,
   mit mindestens einer Fettsäure und/oder deren Ester oder Anhydrid, insbesondere mit mindestens einer C₅-C₃₄-Fettsäure und/oder deren Ester oder Anhydrid, vorzugsweise mit mindestens einer C₈-C₃₄-Fettsäure und/oder deren Ester oder Anhydrid, umgesetzt wird,
   gefolgt von einem zweiten Verfahrensschritt, wobei der zweite Verfahrensschritt

   (i) eine zumindest teilweise Veresterung noch vorhandener Hydroxylgruppen mittels einer Verbindung der allgemeinen Formel (I), wie zuvor definiert, und/oder
   (ii) eine teilweisen Umesterung von im ersten Verfahrensschritt eingeführten Estergruppen mittels einer Verbindung der allgemeinen Formel (I), wie zuvor definiert,
   umfasst;
so dass als Reaktionsprodukt jeweils ein oder mehrere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester erhalten werden.

Insbesondere kann das zuvor beschriebene Herstellungsverfahren gemäß Syntheseroute (A) entsprechend dem zuvor beschriebenen erfindungsgemäßen Verfahren durchgeführt werden.

Was die im Rahmen des zuvor beschriebenen Verfahrens gemäß Syntheseroute (B) einsetzbare Fettsäure und/oder Fettsäureester anbelangt, so ist es insbesondere bevorzugt, wenn die Fettsäure und/oder der Fettsäureester eine Carbonsäure und/oder ein Carbonsäureester der allgemeinen Formel (IX), wie zuvor definiert, ist.

Was weiterhin das im Rahmen des zuvor beschriebenen Verfahrens gemäß Syntheseroute (B) einsetzbare Fettsäureanhydrid anbelangt, so ist es insbesondere bevorzugt, wenn das Fettsäureanhydrid ein Carbonsäureanhydrid der allgemeinen Formel (VII), wie zuvor definiert, ist.

Insbesondere wird bei dem zuvor beschriebenen Verfahren gemäß Syntheseroute (B) im ersten Verfahrensschritt die Umsetzung in Abwesenheit von Lösemitteln und/oder ohne jedwedes Lösemittel durchgeführt. Wie zuvor ausgeführt heißt das, dass die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer besonderen Ausführungsform des zuvor beschriebenen Verfahrens gemäß Syntheseroute (B) kann im ersten Verfahrensschritt im Fall der Verwendung von Fettsäure und/oder deren Ester als Edukt in Gegenwart eines Katalysators, insbesondere eines metallhaltigen und/oder metallbasierten, sauren oder basischen Katalysators, durchgeführt werden.

In diesem Zusammenhang ist es insbesondere bevorzugt, wenn der Katalysators nach Umsetzung rezykliert wird.

Alternativ zu dieser besonderen Ausführungsform wird gemäß Syntheseroute (B) die Umsetzung im Fall der Verwendung von Fettsäureanhydrid als Edukt in Abwesenheit eines Katalysators und/oder ohne einen Katalysator durchgeführt.

Wie zuvor ausgeführt, kann gemäß einer besonderen Ausführungsform des zuvor beschriebenen Herstellungsverfahrens gemäß Syntheseroute (B) im ersten Verfahrensschritt die Umsetzung im Fall der Verwendung von Fettsäure und/oder deren Ester als Edukt in Gegenwart eines metallhaltigen und/oder metallbasierten, sauren oder basischen Katalysators durchgeführt werden.

Dabei kann der Katalysator ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es in diesem Zusammenhang, wie zuvor bereits erwähnt, bevorzugt, wenn der Katalysator nach der Umsetzung rezykliert wird.

Wenn gemäß der besonderen Ausführungsform die Umsetzung in Gegenwart eines metallhaltigen und/oder metallbasierten, sauren oder basischen Katalysators durchgeführt ist, ist es bevorzugt, wenn die Umsetzung bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt wird.

Insbesondere ist es gemäß dieser Ausführungsform auch bevorzugt, wenn der Katalysator in Mengen, bezogen auf die Gesamtmenge der Ausgangsverbindungen, im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird.

Weiterhin ist es gemäß dieser Ausführungsform bevorzugt, wenn die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

Im Rahmen des zuvor beschriebenen Herstellungsverfahrens gemäß Syntheseroute (B) wird im Fall der Verwendung von Fettsäure und/oder deren Ester als Edukt bei der Umsetzung gleichzeitig die Verbindung der allgemeinen Formel (XI), wie zuvor definiert, gebildet.

In diesem Zusammenhang ist es insbesondere bevorzugt, wenn die Verbindung der allgemeinen Formel (XI) während oder nach erfolgter Umsetzung, insbesondere während der Umsetzung, entfernt wird, vorzugsweise destillativ.

Im Rahmen des zuvor beschriebenen Herstellungsverfahrens gemäß Syntheseroute (B) wird im Fall der Verwendung von Fettsäureanhydrid als Edukt bei der Umsetzung gleichzeitig die Verbindung der allgemeinen Formel (VIII), wie zuvor definiert, gebildet.

Dabei ist es insbesondere bevorzugt, wenn die Verbindung der allgemeinen Formel (VIII) während oder nach erfolgter Umsetzung entfernt wird, vorzugsweise destillativ.

Insbesondere wird bei dem zuvor beschriebenen Verfahren gemäß Syntheseroute (B) auch der zweite Verfahrensschritt in Abwesenheit von Lösemitteln und/oder ohne jedwedes Lösemittel durchgeführt.

Weiterhin wird bei dem zuvor beschriebenen Verfahren gemäß Syntheseroute (B) auch der zweite Verfahrensschritt in Gegenwart eines Katalysators, insbesondere eines metallhaltigen und/oder metallbasierten, sauren oder basischen Katalysators, insbesondere wie zuvor definiert, durchgeführt.

Auch in diesem Zusammenhang ist es bevorzugt, wenn der Katalysator nach der Umsetzung rezykliert wird.

Im Rahmen dieser besonderen Ausführungsform des zuvor beschriebenen Verfahrens wird gemäß Syntheseroute (B) im zweiten Verfahrensschritt gleichzeitig die Verbindung der allgemeinen Formel (X)

R¹ - O - R⁵ (X)

gebildet, wobei in der allgemeinen Formel (X)
- der Rest R¹ Wasserstoff oder ein C₁-C₄-Alkyl, insbesondere ein C₁-C₄-Alkyl, vorzugsweise Methyl oder Ethyl, bevorzugt Ethyl, darstellt,
- der Rest R⁵ jeweils unabhängig voneinander, gleich oder verschieden, Wasserstoff oder einen Rest R⁴, wie zuvor definiert, darstellt.

In diesem Zusammen ist es insbesondere vorgesehen, dass die Verbindung der allgemeinen Formel (X) während oder nach erfolgter Umsetzung, insbesondere während der Umsetzung, entfernt wird, vorzugsweise destillativ.

Erfindungsgemäß ist es insbesondere vorgesehen, dass die Fettsäure, vorzugsweise die C₅-C₃₄-Fettsäure, bevorzugt die C₈-C₃₄-Fettsäure, insbesondere in freier Form oder in Form von deren Ester oder Anhydrid, ausgewählt ist aus der Gruppe von Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Heneicosansäure, Behensäure, Lignocerinsäure, Cerotinsäure, Montansäure, Melissinsäure, Laccersäure, Geddinsäure, Undecylsäure, Myristoleinsäure, Palmitoleinsäure, Margaroleinsäure, Petroselinsäure, Ölsäure, Elaidinsäure, Vaccensäure, Gadoleinsäure, Cetoleinsäure, Erucasäure, Nervonsäure, Linolsäure, Linolensäuren, Calendulasäure, Punicinsäure, Eleostearinsäuren, Stearidonsäure, Arachidonsäure, Eicosapentaensäure, Docosadiensäure, Docosatetraensäure, Docosapentaensäure, Docosahexaensäure, und Tetracosahexaensäure sowie deren Mischungen

Gemäß einer besonderen Ausführungsform der vorliegenden Erfindung ist es bevorzugt, wenn die Fettsäure, vorzugsweise die C₅-C₃₄-Fettsäure, bevorzugt die C₈-C₃₄-Fettsäure, insbesondere in freier Form oder in Form von deren Ester oder Anhydrid, ausgewählt ist aus der Gruppe von Myristinsäure, Pentadecansäure, Palmitoleinsäure, Cetoleinsäure, Ölsäure, Gadoleinsäure, Cetoleinsäure, Erucasäure, Arachidonsäure, Eicosapentaensäure, Docosadiensäure, Docosatetraensäure, Docosapentaensäure, Docosahexaensäure, Tetracosahexaensäure sowie deren Mischungen, vorzugsweise Eicosapentaensäure und Docosahexaensäure sowie deren Mischungen.

Gemäß einer weiteren besonderen Ausführungsform ist die Fettsäure, vorzugsweise die C₅-C₃₄-Fettsäure, bevorzugt die C₈-C₃₄-Fettsäure, insbesondere in freier Form oder in Form von deren Ester oder Anhydrid, ausgewählt ist aus der Gruppe von fischölbasierten und/oder in Fischölen vorkommenden Fettsäuren, insbesondere Eicosapentaensäure, Docosadiensäure, Docosatetraensäure, Docosapentaensäure, Docosahexaensäure und Tetracosahexaensäure sowie deren Mischungen, vorzugsweise Eicosapentaensäure, Docosahexaensäure sowie deren Mischungen.

Im Rahmen des erfindungsgemäßen Verfahrens lassen sich als Reaktionsprodukt ein oder mehrere gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, acetoacetylverkappte 3-Hydroxybuttersäure-Polyglycerinester (III') erhalten.

Weiterhin lassen sich im Rahmen des erfindungsgemäßen Verfahrens als Reaktionsprodukt ein oder mehrere acetoacetylverkappte 3-Hydroxybuttersäure-Polyglycerinester erhalten.

Des Weiteren lassen sich im Rahmen des erfindungsgemäßen Verfahrens als Reaktionsprodukt ein oder mehrere funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester (III') erhalten.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt bzw. einen wie zuvor beschriebenen Polyglycerinester der acetoacetylverkappten 3-Hydroxybuttersäure (beta-Hydroxybuttersäure, 3-Hydroxybutansäure) bzw. diesbezügliche Gemische hiervon.

Gemäß diesem Erfindungsaspekt betrifft die vorliegende Erfindung somit einen gegebenenfalls funktionalisierten acetoacetylverkappten 3-Hydroxybuttersäure-Polyglycerinester der allgemeinen Formel (IIIb")

R⁶O - CH₂ - CH(OR⁶) - CH₂ - [O - CH₂ - CH(OR⁶) - CH₂]ₚ - OR⁶ (IIIb")

wobei in der allgemeinen Formel (IIIb")
- die Variable p eine ganze Zahl 1 bis 4, vorzugsweise 1 oder 2, besonders bevorzugt 1, darstellt,
- der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

Gemäß einer besonderen Ausführungsform der vorliegenden Erfindung kann das Reaktionsprodukt ein oder mehrere gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, acetoacetylverkappte 3-Hydroxybuttersäure-Polyglycerinester der allgemeinen Formel (IIIb")

R⁶O - CH₂ - CH(OR⁶) - CH₂ - [O - CH₂ - CH(OR⁶) - CH₂]ₚ - OR⁶ (IIIb")

umfassen,
wobei in der allgemeinen Formel (IIIb")
- die Variable p eine ganze Zahl von 1 bis 4, vorzugsweise 1 oder 2, besonders bevorzugt 1, darstellt,
- der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

Gemäß einer weiteren besonderen Ausführungsform der vorliegenden Erfindung kann das Reaktionsprodukt ein oder mehrere gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, acetoacetylverkappte 3-Hydroxybuttersäure-Polyglycerinester der allgemeinen Formel (IIIc")

R⁶O - CH₂ - CH(OR⁶) - CH₂ - O - CH₂ - CH(OR⁶) - CH₂ - OR⁶ (IIIc")

umfassen,
wobei in der allgemeinen Formel (IIIc") der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

Gemäß einer besonderen Ausführungsform der vorliegenden Erfindung kann das Reaktionsprodukt insbesondere ein Gemisch von mindestens zwei voneinander verschiedenen gegebenenfalls funktionalisierten, vorzugweise gegebenenfalls fettsäurefunktionalisierten, bevorzugt gegebenenfalls C₅-C₃₄-fettsäurefunktionalisierten, insbesondere gegebenenfalls C₈-C₃₄-fettsäurefunktionalisierten, acetoacetylverkappten 3-Hydroxybuttersäure-Polyglycerinestern (III"), insbesondere wie zuvor definiert, umfassen.

Gemäß einer weiteren besonderen Ausführungsform der vorliegenden Erfindung kann das Reaktionsprodukt insbesondere ein Gemisch von mindestens drei voneinander verschiedenen gegebenenfalls funktionalisierten, vorzugsweise gegebenenfalls fettsäurefunktionalisierten, bevorzugt gegebenenfalls C₅-C₃₄-fettsäurefunktionalisierten, insbesondere gegebenenfalls C₈-C₃₄-fettsäurefunktionalisierten, acetoacetylverkappten 3-Hydroxybuttersäure-Polyglycerinestern (III"), insbesondere wie zuvor definiert, umfassen.

Insbesondere kann das Reaktionsprodukt ein oder mehrere acetoacetylverkappte 3-Hydroxybuttersäure-Polyglycerinester der allgemeinen Formel (IIIb)

R⁷O - CH₂ - CH(OR⁷) - CH₂ - [O - CH₂ - CH(OR⁷) - CH₂]ₚ - OR⁷ (IIIb)

umfassen,
wobei in der allgemeinen Formel (IIIb)
- die Variable p eine ganze Zahl von 1 bis 4, vorzugsweise 1 oder 2, besonders bevorzugt 1, darstellt,
- der Rest R¹ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, jedoch mit der Maßgabe, dass mindestens ein Rest R⁷, insbesondere mindestens zwei Reste R⁷, keinen Wasserstoff darstellt.

Gemäß einer besonderen Ausführungsform der vorliegenden Erfindung kann das Reaktionsprodukt ein oder mehrere acetoacetylverkappte 3-Hydroxybuttersäure-Polyglycerinester der allgemeinen Formel (IIIc)

R⁷O - CH₂ - CH(OR⁷) - CH₂ - O - CH₂ - CH(OR⁷) - CH₂ - OR⁷ (IIIc)

umfassen,
wobei in der allgemeinen Formel (IIIc) der Rest R⁷ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, jedoch mit der Maßgabe, dass mindestens ein Rest R⁷, insbesondere mindestens zwei Reste R⁷, keinen Wasserstoff darstellt.

Gemäß einer besonderen Ausführungsform kann das Reaktionsprodukt insbesondere ein Gemisch von mindestens zwei voneinander verschiedenen acetoacetylverkappten 3-Hydroxybuttersäure-Polyglycerinestern, insbesondere wie zuvor definiert, umfassen.

Gemäß einer weiteren besonderen Ausführungsform kann das Reaktionsprodukt insbesondere ein Gemisch von mindestens drei voneinander verschiedenen acetoacetylverkappten 3-Hydroxybuttersäure-Polyglycerinestern, insbesondere wie zuvor definiert, umfassen.

Insbesondere kann das Reaktionsprodukt ein oder mehrere funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester der allgemeinen Formel (IIIb')

R⁶O - CH₂ - CH(OR⁶) - CH₂ - [O - CH₂ - CH(OR⁶) - CH₂]ₚ - OR⁶ (IIIb')

umfassen,
wobei in der allgemeinen Formel (IIIb')
- die Variable p eine ganze Zahl von 1 bis 4, vorzugsweise 1 oder 2, besonders bevorzugt 1, darstellt,
- der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, einen Rest CH₃ - CH(OR²) - CH₂-C(O) -, wie zuvor definiert, darstellt, und mit der Maßgabe, dass mindestens ein Rest R⁶, insbesondere mindestens zwei Reste R⁶, einen Rest R⁴, wie zuvor definiert, darstellt.

Insbesondere kann in der allgemeinen Formel (IIIb') der Rest R⁶ jeweils unabhängig voneinander, gleich oder verschieden, darstellen: einen Rest CH₃-CH(OR²) - CH₂ - C(O) -, wie zuvor definiert, oder einen Rest R⁴, wie zuvor definiert, jedoch mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶ einen Rest R⁴, wie zuvor definiert, darstellt.

Dabei ist es insbesondere bevorzugt, wenn in der allgemeinen Formel (IIIb') der Rest R⁶ jeweils unabhängig voneinander, gleich oder verschieden, keinen Wasserstoff darstellt.

Gemäß einer besonderen Ausführungsform der vorliegenden Erfindung kann das Reaktionsprodukt ein oder mehrere funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester der allgemeinen Formel (IIIc')

R⁶O - CH₂ - CH(OR⁶) - CH₂ - O - CH₂ - CH(OR⁶) - CH₂-OR⁶ (IIIc')

umfassen,
wobei in der allgemeinen Formel (IIIc') der Rest R⁶ jeweils unabhängig voneinander, gleich oder verschieden, darstellt: Wasserstoff oder einen Rest CH₃-CH(OR²) - CH₂ - C(O) -, wobei der Rest R² einen Rest CH₃ - C(O) - CH₂-C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O)- insbesondere (C₄-C₃₃-Alkyl) - C(O) -, vorzugsweise (C₇-C₃₃-Alkyl) - C(O) -, darstellt, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶, insbesondere mindestens zwei Reste R⁶, einen Rest R⁴, wie zuvor definiert, darstellt.

Insbesondere kann in der allgemeinen Formel (IIIc') der Rest R⁶ jeweils unabhängig voneinander, gleich oder verschieden, darstellen: einen Rest CH₃-CH(OR²) - CH₂ - C(O) -, wie zuvor definiert, oder einen Rest R⁴, wie zuvor definiert, jedoch mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶ einen Rest R⁴, wie zuvor definiert, darstellt.

Dabei ist es insbesondere bevorzugt, wenn in der allgemeinen Formel (IIIc') der Rest R⁶ jeweils unabhängig voneinander, gleich oder verschieden, keinen Wasserstoff darstellt.

Gemäß einer besonderen Ausführungsform kann das Reaktionsprodukt insbesondere ein Gemisch von mindestens zwei voneinander verschiedenen funktionalisierten, insbesondere fettsäurefunktionalisierten, vorzugsweise C₅-C₃₄-fettsäurefunktionalisierten, bevorzugt C₈-C₃₄-fettsäurefunktionalisierten, acetoacetylverkappten 3-Hydroxybuttersäure-Polyglycerinestern (III'), insbesondere wie zuvor definiert, umfassen.

Gemäß einer weiteren besonderen Ausführungsform kann das Reaktionsprodukt insbesondere ein Gemisch von mindestens drei voneinander verschiedenen funktionalisierten, insbesondere fettsäurefunktionalisierten, vorzugsweise C₅-C₃₄-fettsäurefunktionalisierten, bevorzugt C₈-C₃₄-fettsäurefunktionalisierten, acetoacetylverkappten 3-Hydroxybuttersäure-Polyglycerinestern (III'), insbesondere wie zuvor definiert, umfassen.

Weiterer Gegenstand der vorliegenden Erfindung gemäß diesem Erfindungsaspekt ist - wie bereits zuvor dargelegt - ebenfalls ein gegebenenfalls funktionalisierter acetoacetylverkappter 3-Hydroxybuttersäure-Polyolester (III"), insbesondere wie zuvor beschrieben,
wobei der gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, acetoacetylverkappte 3-Hydroxybuttersäure-Polyglycerinester der allgemeinen Formel (IIIb")

   R⁶O - CH₂ - CH(OR⁶) - CH₂ - [O - CH₂ - CH(OR⁶) - CH₂]ₚ - OR⁶ (IIIb")

   entspricht,
wobei in der allgemeinen Formel (IIIb")
   - die Variable p eine ganze Zahl von 1 bis 4, vorzugsweise 1 oder 2, besonders bevorzugt 1, darstellt,
   - der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

Wiederum weiterer Gegenstand der vorliegenden Erfindung ist ebenfalls ein gegebenenfalls funktionalisierter acetoacetylverkappter 3-Hydroxybuttersäure-Polyolester (III"), insbesondere wie zuvor beschrieben,
wobei der gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, acetoacetylverkappte 3-Hydroxybuttersäure-Polyglycerinester der allgemeinen Formel (IIIc")

   R⁶O - CH₂ - CH(OR⁶) - CH₂ - O - CH₂ - CH(OR⁶) - CH₂-OR⁶ (IIIc")

   entspricht,
wobei in der allgemeinen Formel (IIIc") der Rest R⁶ jeweils unabhängig voneinander, gleich oder verschieden, darstellt: Wasserstoff oder einen Rest CH₃-CH(OR²) - CH₂ - C(O) -, wobei der Rest R² einen Rest CH₃ - C(O) - CH₂-C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂-C(O) -, wie zuvor definiert, darstellt.

Weiterer Gegenstand der vorliegenden Erfindung gemäß diesem Erfindungsaspekt ist entsprechend einer besonderen Ausführungsform ein Gemisch, welches mindestens zwei voneinander verschiedene gegebenenfalls funktionalisierteacetoacetylverkappte 3-Hydroxybuttersäure-Polyglycerinester (III'), wie zuvor definiert, umfasst.

Wiederum weiterer Gegenstand der vorliegenden Erfindung gemäß diesem Erfindungsaspekt ist entsprechend einer besonderen Ausführungsform ein Gemisch, welches mindestens drei voneinander verschiedene gegebenenfalls funktionalisierteacetoacetylverkappte 3-Hydroxybuttersäure-Polyglycerinester (III"), insbesondere wie zuvor definiert, umfasst.

Gemäß dieser Ausführungsform ist ein weiterer Gegenstand der vorliegenden Erfindung ein acetoacetylverkappter 3-Hydroxybuttersäure-Polyolester, insbesondere wie zuvor beschrieben,
wobei der acetoacetylverkappte 3-Hydroxybuttersäure-Polyglycerinester der allgemeinen Formel (IIIb)

   R⁷O - CH₂ - CH(OR⁷) - CH₂ - [O - CH₂ - CH(OR⁷) - CH₂]ₚ - OR⁷ (IIIb)

   entspricht,
wobei in der allgemeinen Formel (IIIb)

- die Variable p eine ganze Zahl von 1 bis 4, vorzugsweise 1 oder 2, besonders bevorzugt 1, darstellt,
- der Rest R⁷ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, jedoch mit der Maßgabe, dass mindestens ein Rest R⁷, insbesondere mindestens zwei Reste R⁷, keinen Wasserstoff darstellt.

Gemäß dieser Ausführungsform ist ein wiederum weiterer Gegenstand der vorliegenden Erfindung ein acetoacetylverkappter 3-Hydroxybuttersäure-Polyolester, insbesondere wie zuvor beschrieben,
wobei der acetoacetylverkappte 3-Hydroxybuttersäure-Polyglycerinester der allgemeinen Formel (IIIc)

   R⁷O - CH₂ - CH(OR⁷) - CH₂ - O - CH₂ - CH(OR⁷) - CH₂ - OR⁷ (IIIc)

   entspricht,
wobei in der allgemeinen Formel (IIIc) der Rest R⁷ jeweils unabhängig voneinander, gleich oder verschieden, darstellt: Wasserstoff oder einen Rest CH₃-CH(OR²) - CH₂ - C(O) -, wobei der Rest R² einen Rest CH₃ - C(O) - CH₂-C(O) - darstellt, jedoch mit der Maßgabe, dass mindestens ein Rest R⁷, insbesondere mindestens zwei Reste R⁷, keinen Wasserstoff darstellt.

Ein weiterer Gegenstand der vorliegenden Erfindung gemäß diesem Erfindungsaspekt entsprechend einer weiteren besonderen Ausführungsform ist ein Gemisch, welches mindestens zwei voneinander verschiedene acetoacetylverkappte 3-Hydroxybuttersäure-Polyglycerinester, insbesondere wie zuvor definiert, umfasst.

Ein wiederum weiterer Gegenstand der vorliegenden Erfindung gemäß diesem Erfindungsaspekt entsprechend einer weiteren besonderen Ausführungsform ist ein Gemisch, welches mindestens drei voneinander verschiedene acetoacetylverkappte 3-Hydroxybuttersäure-Polyglycerinester, insbesondere wie zuvor definiert, umfasst.

Gemäß dieser besonderen Ausführungsform ist ein weiterer Gegenstand der vorliegenden Erfindung ein funktionalisierter acetoacetylverkappter 3-Hydroxybuttersäure-Polyolester (III'), insbesondere wie zuvor beschrieben,
wobei der funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester der allgemeinen Formel (IIIb')

   R⁶O - CH₂ - CH(OR⁶) - CH₂ - [O - CH₂ - CH(OR⁶) - CH₂]ₚ - OR⁶ (IIIb')

   entspricht,
wobei in der allgemeinen Formel (IIIb')
   - die Variable p eine ganze Zahl von 1 bis 4, vorzugsweise 1 oder 2, besonders bevorzugt 1, darstellt,
   - der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, einen Rest CH₃ - CH(OR²) -CH₂-C(O) -, wie zuvor definiert, darstellt, und mit der Maßgabe, dass mindestens ein Rest R⁶, insbesondere mindestens zwei Reste R⁶, einen Rest R⁴, wie zuvor definiert, darstellt.

Insbesondere kann in der allgemeinen Formel (IIIb') der Rest R⁶ jeweils unabhängig voneinander, gleich oder verschieden, darstellen: einen Rest CH₃ -CH(OR²) - CH₂ - C(O) -, wie zuvor definiert, oder einen Rest R⁴, wie zuvor definiert, jedoch mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶ einen Rest R⁴, wie zuvor definiert, darstellt.

Bei dieser besonderen Ausführungsform ist es bevorzugt, wenn in der allgemeinen Formel (IIIb') der Rest R⁶ jeweils unabhängig voneinander, gleich oder verschieden, keinen Wasserstoff darstellt.

Gemäß dieser besonderen Ausführungsform ist ein weiterer Gegenstand der vorliegenden Erfindung ein funktionalisierter acetoacetylverkappter 3-Hydroxybuttersäure-Polyolester (III'), insbesondere wie zuvor beschrieben, wobei der funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester der allgemeinen Formel (IIIc')

R⁶O - CH₂ - CH(OR⁶) - CH₂ - O - CH₂ - CH(OR⁶) - CH₂ - OR⁶ (IIIc')

entspricht,
wobei in der allgemeinen Formel (IIIc') der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶, insbesondere mindestens zwei Reste R⁶, einen Rest R⁴, wie zuvor definiert, darstellt.

Insbesondere kann in der allgemeinen Formel (IIIc') der Rest R⁶ jeweils unabhängig voneinander, gleich oder verschieden, darstellen: einen Rest CH₃ -CH(OR²) - CH₂ - C(O) -, wie zuvor definiert, oder einen Rest R⁴, wie zuvor definiert, jedoch mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt, und mit der Maßgabe, dass mindestens ein Rest R⁶ einen Rest R⁴, wie zuvor definiert, darstellt.

Bei dieser besonderen Ausführungsform ist es bevorzugt, wenn in der allgemeinen Formel (IIIc') der Rest R⁶ jeweils unabhängig voneinander, gleich oder verschieden, keinen Wasserstoff darstellt.

Weiterer Gegenstand der vorliegenden Erfindung gemäß diesem Erfindungsaspekt ist entsprechend einer weiteren besonderen Ausführungsform ein Gemisch, welches mindestens zwei voneinander verschiedene funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester (III'), insbesondere wie zuvor definiert, umfasst.

Wiederum weiterer Gegenstand der vorliegenden Erfindung gemäß diesem Erfindungsaspekt ist entsprechend einer weiteren besonderen Ausführungsform ein Gemisch, welches mindestens drei voneinander verschiedene funktionalisierte, insbesondere fettsäurefunktionalisierte, vorzugsweise C₅-C₃₄-fettsäurefunktionalisierte, bevorzugt C₈-C₃₄-fettsäurefunktionalisierte, acetoacetylverkappte 3-Hydroxybuttersäure-Polyglycerinester (III'), insbesondere wie zuvor definiert, umfasst.

Wie die Anmelderin überraschend herausgefunden hat, eignet sich der nach dem erfindungsgemäßen Herstellungsverfahren erhältliche bzw. erfindungsgemäße gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieses bzw. diese einerseits physiologisch, insbesondere im Magen/Darm-Trakt, zu den Ketokörpern 3-Hydroxybuttersäure und 3-Oxobutyrat (= Acetoacetat bzw. Acetacetat), welches physiologisch letztlich zu 3-Hydroxybuttersäure bzw. deren Salzen umgesetzt bzw. reduziert wird, gespalten wird und andererseits gleichzeitig eine gute physiologische Kompatibilität bzw. Verträglichkeit aufweist, insbesondere im Hinblick auf Nichttoxizität und akzeptable organoleptische Eigenschaften. Insbesondere die retardierte Freisetzung des physiologisch wirksamen Stoffes im Magen/Darm-Trakt ist im medizinischen Bereich vorteilhaft, da der Wirkstoff 3-Hydroxybuttersäure so über einen längeren Zeitraum zur Verfügung gestellt werden kann und somit eine Ketose-Therapie ermöglicht wird.

Daher eignen sich der nach dem erfindungsgemäßen Herstellungsverfahren erhältliche bzw. erfindungsgemäße gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, als wirksame Präkursoren oder Metabolite, welche physiologisch einen direkten oder indirekten Zugang zu 3-Hydroxybuttersäure oder deren Salzen sowie zu Acetoacetat (und damit physiologisch wiederum zu 3-Hydroxybuttersäure oder deren Salzen) ermöglichen, insbesondere im physiologischen Stoffwechsel des menschlichen oder tierischen Körpers.

Bei der physiologischen Spaltung im Magen und/oder Darm wird also der nach dem erfindungsgemäßen Herstellungsverfahren erhältliche bzw. erfindungsgemäße gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, in die Ketoverbindungen 3-Hydroxybuttersäure und 3-Oxobutyrat (Acetoacetat bzw. Acetacetat), welches vom Körper weiter zu 3-Hydroxybutyrat reduziert werden kann, gespalten.

Durch die Anwesenheit von sowohl 3-Oxobutyratresten als auch 3-Hydroxybutyratresten bzw. 3-Hydroxybuttersäure liegt eine unterschiedlich schnelle Verfügbarkeit bzw. Freisetzung des Wirkstoffs 3-Hydroxybuttersäure vor. Das erfindungsgemäße Reaktionsprodukt weist folglich einen intrinsischen, in sich nochmals differenzierten Retard-Effekt auf. Denn insgesamt weist der erfindungsgemäße gegebenenfalls funktionalisierte Polyglycerinester von acetoacetylverkappter 3-Hydroxybuttersäure somit zwei Ketokörper mit unterschiedlich schnellem Abbau auf. Eine zusätzliche bzw. weiterführende Retardierung liegt zudem dadurch vor, dass die Ketokörper in Form eines Polyolesters vorliegen und somit zur Freisetzung der Wirkstoffe 3-Hydroxybuttersäure in freier Form und Acetoacetat zusätzlich eine Abspaltung vom Polyol erfolgen muss. Insgesamt werden die Wirkstoffe 3-Hydroxybuttersäure und Acetoacetat aus dem erfindungsgemäßen gegebenenfalls funktionalisierten Polyglycerinester von acetoacetylverkappter 3-Hydroxybuttersäure durch einen mehrstufigen Abbau mit Retardierungseffekt freigesetzt.

Das erfindungsgemäße Verfahren ermöglicht somit erstmalig die Bereitstellung untoxischer, gegebenenfalls funktionalisierter Polyglycerinester von acetoacetylverkappter 3-Hydroxybuttersäure aus an sich bekannten, kommerziell verfügbaren und vor allem physiologisch unbedenklichen Komponenten bzw. Edukten (Ausgangsverbindungen). Die resultierenden gegebenenfalls funktionalisierten Polyglycerinester von acetoacetylverkappter 3-Hydroxybuttersäure können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren. Gleichzeitig wird auch der weitere Ketokörper Acetoacetat aus der Acylverkappung der 3-Hydroxybuttersäure physiologisch freigesetzt (welcher dann weiter zur 3-Hydroxybuttersäure reduziert wird).

Darüber hinaus ist der nach dem erfindungsgemäßen Herstellungsverfahren erhältliche bzw. erfindungsgemäße gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch im großtechnischen Maßstab zugänglich bzw. verfügbar, und zwar auch mit der erforderlichen pharmazeutischen bzw. pharmakologischen Qualität.

Zudem kann der gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, in einer enantiomerenreiner bzw. enantiomerenangereicherten Form bereitgestellt werden.

Der nach dem erfindungsgemäßen Herstellungsverfahren erhältliche bzw. erfindungsgemäße gegebenenfalls funktionalisierte Polyglycerinester von acetoacetylverkappter 3-Hydroxybuttersäure, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführend erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältlichen bzw. erfindungsgemäßen gegebenenfalls funktionalisierten Polyglycerinester von acetoacetylverkappter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Auch eignet sich ein nach dem erfindungsgemäßen Herstellungsverfahren erhältlicher bzw. erfindungsgemäßer gegebenenfalls funktionalisierter Polyglycerinester von acetoacetylverkappter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **vierten** Aspekt der vorliegenden Erfindung - ist die Verwendung mindestens eines gegebenenfalls funktionalisierten Polyglycerinesters von acetoacetylverkappter 3-Hydroxybuttersäure, wie zuvor definiert, und/oder Verwendung eines Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Darüber hinaus kann die Verwendung eines nach dem erfindungsgemäßen Herstellungsverfahren erhältlichen bzw. erfindungsgemäßen gegebenenfalls funktionalisierten Polyglycerinesters von acetoacetylverkappter 3-Hydroxybuttersäure oder deren Salz oder Ester, wie zuvor definiert, und/oder Verwendung eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung, vorgesehen sein.

Gleichermaßen weiterer Gegenstand der vorliegenden Erfindung - gemäß einem **fünften** Aspekt der vorliegenden Erfindung - ist ein Nahrungsmittel- und/oder Lebensmittelerzeugnis, welches ein nach dem erfindungsgemäßen Herstellungsverfahren erhältlichen bzw. erfindungsgemäßen gegebenenfalls funktionalisierten Polyglycerinester von acetoacetylverkappter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **sechsten** Aspekt der vorliegenden Erfindung - die Verwendung eines nach dem erfindungsgemäßen Herstellungsverfahren erhältlichen bzw. erfindungsgemäßen gegebenenfalls funktionalisierten Polyglycerinesters von acetoacetylverkappter 3-Hydroxybuttersäure, wie zuvor definiert, und/oder eines nach dem erfindungsgemäßen Herstellungsverfahren erhältlichen bzw. erfindungsgemäßen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- 3-BHB = 3-Hydroxybuttersäure bzw. 3-Hydroxybuttersäure-Rest (3-Hydroxybutyrat-Rest)
- 3-BHB-FS = 3-Hydroxybuttersäure (freie Säure)
- PG(2) = Diglycerin: HO - CH₂ - CH(OH) - CH₂ - O - CH₂ - CH(OH) - CH₂- OH
- PG(3) = Polyglycerin: HO - CH₂ - CH(OH) - CH₂ - [O - CH₂ - CH(OH) - CH₂]₂ - OH
- 3-BHB-Dimer-Ethylester = Dimer des 3-BHB-Ethylesters
- 3-Acetylaceto-BHB₂-Ethylester = mit Ethylacetoacetat verkapptes Dimer des 3-BHB-Ethylesters

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Herstellung von 3-Acetylacetobuttersäure-Diglycerinester-Gemischen

In einem 100-ml-Mehrhalskolben mit Dephlegmator (Partialkondensator) und Destillationsbrücke werden 89 g 3-Acetylacetobuttersäureethylester (3-Acetylaceto-BHB-Ethylester), 3,5 g Diglycerin und 0,9 g immobilisiertes Enzym (CALB-Lipase auf Polymerträger, abgeleitet von *Candida antarctica,* z. B. Novozym^{®} 435) vorgelegt.

Das Reaktionsgemisch wird bei 50 °C bis 70 °C unter Vakuum und Rühren für 6 h zur Reaktion gebracht. Das entstehende Ethanol wird kontinuierlich abdestilliert. Anschließend wird das Enzym abfiltriert und überschüssiger 3-Acetylacetobuttersäureethylester unter Vakuum abdestilliert. Es wird ein Gemisch aus Mono-, Di-, Tri- und Tetra-Diglycerinestern der 3-Acetylacetobuttersäure erhalten. Die Charakterisierung erfolgt mittels Gaschromatohraphie (GC), Gelpermeationschromatographie (GPC) und GC-MS-Analyse (Gaschromatographie mit Massenspektrometrie-Kopplung).

Im Rahmen der Aufreinigung werden gegebenenfalls noch vorhandene Edukte und gegebenenfalls vorhandene Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner Mono-3-Acetylacetobuttersäure-Diglycerinester, reiner Di-3-Acetylacetobuttersäure-Diglycerinester, reiner Tri-3-Acetylacetobuttersäure-Diglycerinester und reiner Tetra-3-Acetylacetobuttersäure-Diglycerinester). Ein anderer Teil des Gemischs wird einer Auftrennung mittels fraktionierter Destillation unterzogen.

### Weitere Herstellung von 3-Acetylacetobuttersäure-Diglycerinestern (nicht erfindungsgemäß)

In einem 100-ml-Mehrhalskolben mit Dephlegmator (Partialkondensator) und Destillationsbrücke werden 178 g 3-Acetylacetobuttersäureethylester (3-Acetylaceto-BHB-Ethylester) und 29 g Diglycerin vorgelegt.

Bei einer Temperatur von 100 °C werden 1,4 g 30 %-ige methanolische NaOMe-Lösung unter Rühren zugegeben. Das entstehende Ethanol wird kontinuierlich abdestilliert. Nach einer Reaktionszeit von 5 h wird das Reaktionsgemisch abgekühlt und mit NaCl-Lösung gewaschen. Die Rohestermischung wird anschließend getrocknet und der überschüssige 3-Acetylacetobuttersäureethylester unter Vakuum abdestilliert.

Das Reaktionsprodukt ist ein 3-Acetylacetobuttersäure-Diglycerin-Ester-Gemisch mit folgender Zusammensetzung: Mono-3-Acetylacetobuttersäure-Diglycerinester, Di-3-Acetylacetobuttersäure-Diglycerinester, Tri-3-Acetylacetobuttersäure-Diglycerinester und Tetra-3-Acetylacetobuttersäure-Diglycerinester. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner Mono-3-Acetylacetobuttersäure-Diglycerinester, reiner Di-3-Acetylacetobuttersäure-Diglycerinester, reiner Tri-3-Acetylacetobuttersäure-Diglycerinester etc.). Ein anderer Teil des Gemischs wird einer Auftrennung mittels fraktionierter Destillation unterzogen.

### Weitere Herstellung von acylverkappten 3-BHB-Polyolestern

Die vorangehenden Versuche werden jeweils (mit Enzym und mit NaOMe (nicht erfindungsgemäß) als Katalysator) wiederholt, jedoch mit anderen Polyolen (nämlich mit Glycerin (nicht erfindungsgemäß), Polyglycerin PG(3) und 1,2-Pentandiol (nicht erfindungsgemäß)). Es werden vergleichbare Ergebnisse erhalten. Aufreinigung bzw. Fraktionierung erfolgen in gleicher Weise.

### Nochmals weitere Herstellungsbeispiele

Es werden verschiedene Polyol-Komponenten auf Basis mehrwertiger Alkohole (Polyole) mit 3-Acetylacetobuttersäureethylester enzymatisch umgesetzt.

Als Polyole werden 1,2-Pentandiol (nicht erfindungsgemäß) und Diglycerin PG(2) ausgewählt. Die jeweiligen Polyole werden bei 70 °C für 24 h mit immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) umgesetzt (jeweils 1 Gew.-% Enzym und jeweils 40 Mol-% Überschuss 3-Acetylacetobuttersäureethylester).

Die vorgenannten Polyole 1,2-Pentandiol und Diglycerin PG(2) werden mit den vorgenannten Enzymen effizient zu den gewünschten Produkten umgesetzt. Es werden vergleichbare Ergebnisse zu den vorangehenden Versuchen erhalten. Aufreinigung und Trennung bzw. Fraktionierung erfolgen in gleicher Weise.

Die Versuche werden mit Natriummethanolat (NaOMe) (nicht erfindungsgemäß) als Katalysator anstelle der Enzyme und bei Temperaturen zwischen 100 und 120 °C wiederholt. Es werden vergleichbare Ergebnisse erhalten. Aufreinigung und Trennung bzw. Fraktionierung erfolgen in gleicher Weise.

Da insbesondere die 3-Acetylacetobuttersäure-PG(2)-Ester ein nur wenig bitteren Geschmack aufweisen, sind vor allem diese Ester eine effiziente Produktgruppe für eine therapeutische Anwendung. Daher wird der vorangehende Versuch mit Enzym und Diglycerin PG(2) als Polyol in einem größeren Maßstab (2 bis 4 kg) durchgeführt.

Zunächst werden in einem Maßstab von 2 kg die stöchiometrischen Reaktionsbedingungen der vorangehenden Versuche angewendet (40 Mol-% Überschuss 3-Acetylacetobuttersäureethylester, 1 Gew.-% Enzym). Nach 15 h wird ein Teil der Reaktionsmischung (ca. 200 g) für eine weitere Untersuchung entnommen. Hierbei handelt es sich um ein Mono/Di-PG(2)-Ester-Gemisch. Danach werden weitere ca. 1 kg 3-Acetylacetobuttersäureethylester hinzugegeben.

Das Ziel ist die Herstellung eines Voll-Esters. Es ist zu erkennen, dass sich nach etwa 20 bis 30 h ein konstanter Gehalt an Di-PG(2)-Ester einstellt; der Mono-PG(2)-Ester Anteil fällt ab und der Tri-PG(2)-Ester-Anteil steigt. Weitere Analysen (GPC) zeigen, dass sich auch ein Tetra-PG(2)-Ester gebildet hat.

Nach Abdestillieren von überschüssigem 3-Acetylacetobuttersäure-Ethylester weist das zunächst erhaltene (niedrigsiedende) Mono/Di-PG(2)-Ester-Gemisch nur einen leicht bitteren Geschmack auf, während das höhere (höhersiedende) Di-/Tri-/Tetra-PG(2)-Ester-Gemisch einen etwas stärker bitteren Geschmack aufweist. Beide Gemische sind jedoch organoleptisch akzeptabel und kompatibel.

Nach weitergehender Aufreinigung unter Entfernung restlicher Edukte und Reaktionsnebenprodukte wird ein Reingemisch mit signifikant verbesserten organoleptischen Eigenschaften erhalten.

### Funktionalisierungsversuche

### 1. Herstellung des Anhydrids

In einem 2.000-ml-Mehrhalskolben mit Dephlegmator (Partialkondensator) und Destillationsbrücke werden 860 g Heptansäure vorgelegt und bei 90 °C werden 445 g Essigsäureanhydrid unter Rühren zugetropft. Das Reaktionsgemisch wird bei 130 °C unter Rückfluss für 6 h gerührt. Anschließend werden die entstandene Essigsäure sowie das überschüssige Essigsäureanhydrid unter Vakuum abdestilliert. Es wird ein Heptansäure/ Heptansäureanhydrid-Gemisch mit folgender Zusammensetzung erhalten: 15 % Heptansäure, 85 % Heptansäureanhydrid. Die Charakterisierung erfolgt mittels GC und GC-MS.

### 2. Funktionalisierung

In einem 100-ml-Mehrhalskolben mit Dephlegmator (Partialkondensator) und Destillationsbrücke werden 25 g des Heptansäureanhydrids und 5 g eines erfindungsgemäß hergestellten 3-Acetylaceto-BHB-Mono-, Di-, Tri-, Tetra-Diglycerin-Gemischs vorgelegt. Das Reaktionsgemisch wird bei 70 °C für 24 h gerührt. Danach werden überschüssiges Heptansäureanhydrid und die entstandene Heptansäure mittels einer Kurzweg-Destillation abdestilliert. Es wird ein 3-Acetylaceto-BHB/Heptansäure-Diglycerinester-Gemisch erhalten (d. h. mit anderen Worten ein an den freien OH-Gruppen heptansäurefunktionalisiertes bzw. heptansäureverestertes 3-Acetylaceto-BHB-Diglycerinester-Gemisch).

Vergleichbare Funktionalisierungsversuche werden auch mit Fettsäuren und alternativ Fettsäureanhydriden und wiederum alternativ Fettsäureestern (jeweils mit Eicosapentaensäure/Docosahexaensäure-Gemisch und deren Anhydriden und Estern einerseits sowie mit Ölsäure und deren Anhydrid und Ester andererseits) durchgeführt und führen jeweils zu analogen Ergebnissen (d. h. Veresterung der freien OH-Gruppen), wie durch entsprechende Analytik bestätigt.

Die Versuche zeigen, dass die beabsichtigte Funktionalisierung (Veresterung) durch Umsetzung mit Carbonsäuren und alternativ Carbonsäureanhydriden und wiederum alternativ Carbonsäureestern zu den gewünschten Produkten führt (d. h. Veresterung der freien OH-Gruppen), wie durch entsprechende Analytik bestätigt.

### Weitere Synthesen funktionalisierter 3-Acetylaceto-BHB-Polyolester (nicht erfindungsgemäß)

Als Polyole werden 1,2-Pentandiol und Diglycerin PG(2) eingesetzt. Die jeweiligen Polyole werden zunächst mit Natriummethanolat (NaOMe) als Katalysator und bei Temperaturen zwischen 100 und 120 °C mit Fettsäuren und alternativ Fettsäureanhydriden und wiederum alternativ Fettsäureestern (jeweils mit Eicosapentaensäure, Docosahexaensäure und Ölsäure und deren Anhydriden und Estern) umgesetzt; es resultieren die entsprechenden fettsäureveresterten Polyole, welche in einem zweiten, nachfolgenden Verfahrensschritt weiter mit 3-Acetylaceto-BHB-Ethylester umgesetzt werden. Es resultieren die entsprechenden 3-Acetylaceto-BHB/Fettsäure-Polyolester-Gemische. Vergleichbare Ergebnisse werden bei umgekehrter Vorgehensweise erhalten (d. h. zunächst Umsetzung der Polyole mit 3-Oxobuttersäureethylester, gefolgt von einer weiteren Umsetzung mit den vorgenannten Fettsäuren bzw. alternativ deren Anhydriden und Estern).

### Herstellung von 3-Acetylaceto-BHB-Ethylester (= Edukt)

In einem 100-ml-Mehrhalskolben mit Dephlegmator (Partialkondensator) und Destillationsbrücke werden 52 g 3-Oxobuttersäureethylester (Ethylacetoacetat bzw. Acetessigester) und 26 g 3-Hydroxybuttersäureethylester vorgelegt.

Bei einer Temperatur von 50 °C und unter Vakuum werden 0,8 g immobilisiertes Enzym (CALB-Lipase auf Polymerträger, abgeleitet von *Candida antarctica,* z. B. Novozym^{®} 435) zugegeben. Das Reaktionsgemisch wird unter Rühren für 6 h zur Reaktion gebracht. Das während der Reaktion entstehende Ethanol wird kontinuierlich abdestilliert. Anschließend wird das Enzym abfiltriert und überschüssiger 3-Oxobuttersäureethylester sowie überschüssiger 3-Hydroxybuttersäureethylester unter Vakuum abdestilliert und rezykliert.

Das erhaltene Reaktionsprodukt ist ein 3-Acetylacetobuttersäureethylester (3-Acetylaceto-BHB-Ethylester) und besteht nach analytischer Untersuchung aus folgender Zusammensetzung: > 90 % 3-Acetylaceto-BHB-Ethylester (Reaktionsnebenprodukte: 3-BHB-Dimer-Ethylester < 5 % und Acetylaceto-BHB-Dimer-Ethylester < 5 %). Destillative Aufreinigung führt zu reinen 3-Acetylacetobuttersäureethylestern (Reinheit > 99,9 %). Die Charakterisierung erfolgt mittels GC und GC-MS.

### Weitere Herstellung von 3-Acetylaceto-BHB-Ethylester (= Edukt)

In einem 100-ml-Mehrhalskolben mit Dephlegmator (Partialkondensator) und Destillationsbrücke werden 30 g 3-Oxobuttersäureethylester (Ethylacetoacetat bzw. Acetessigester) und 15,25 g 3-Hydroxybuttersäureethylester (3-BHB-Ethylester) vorgelegt.

Bei einer Temperatur von 50 °C und unter Vakuum werden 0,46 g immobilisiertem Enzym (CALB-Lipase auf Polymerträger, abgeleitet von *Candida antarctica,* z. B. Novozym^{®} 435) zugegeben. Das Reaktionsgemisch wird unter Rühren für 6 h zur Reaktion gebracht. Das während der Reaktion entstehende Ethanol wird kontinuierlich abdestilliert. Anschließend wird das Enzym abfiltriert und überschüssiger 3-Oxobuttersäureethylester sowie überschüssiger 3-Hydroxybuttersäureethylester unter Vakuum abdestilliert und anschließend rezykliert.

Die Charakterisierung erfolgt mittels GC und GC-MS.

### Wiederum weitere Herstellungsbeispiele von 3-Acetylaceto-BHB-Ethylester (= Edukt)

In einer weiteren Versuchsreihe wird jeweils der Einfluss des Molverhältnisses der Ausgangsverbindungen im Hinblick auf die Bildung von Nebenprodukten (analytisch untersucht anhand der beiden Nebenprodukte "3-BHB-Dimer-Ethylester" und "Acetylaceto-BHB₂-Ethylester") untersucht.

Es zeig sich, dass ein molarer Überschuss an 3-Oxobuttersäureethylester (Ethylacetoacetat bzw. Acetessigester) in Bezug auf das weitere Edukt 3-Hydroxybuttersäureethylester (3-BHB-Ethylester) einer Nebenproduktbildung entgegenwirkt.

In einer ersten Untersuchungsreihe zeigt sich ein Acetessigester / 3-BHB-Ethylester-Molverhältnis im Bereich von 1,5 : 1 bis 9: 1 besonders effizient in Bezug auf eine Nebenproduktbildung und ist auch noch verfahrensökonomisch. Besonders gute Ergebnisse werden in einer zweiten Untersuchungsreihe für ein Acetessigester / 3-BHB-Ethylester-Molverhältnis im Bereich von 2 : 1 bis 8 : 1 beobachtet.

### Physiologische Anwendungsversuche: in-vitro-Verdauversuche

### Verdauversuche (Spalt- bzw. Spaltungsversuche) von erfindungsgemäßen Acylverkappten 3-Hydroxybuttersäure-PG(2)-Ester-Gemischen

Mittels Spaltungsversuchen wird gezeigt, dass erfindungsgemäß hergestellte acetoacetylverkappte 3-Hydroxybuttersäure-PG(2)-Ester bzw. deren Gemische, sowie deren funktionalisierte (d. h. an dreien OH-Gruppen veresterte) Derivate einschließlich der Reaktionsnebenprodukte, im menschlichen gastrointestinalen Trakt gespalten werden können.

Als Ausgangsgemisch wird einerseits ein aufgereinigtes, nach dem erfindungsgemäßen Verfahren erhaltenes Gemisch aus Mono-3-Acetylaceto-BHB-Diglycerinester, Di-3-Acetylaceto-BHB-Diglycerinester, Tri-3-Acetylaceto-BHB-Diglycerinester und Tetra-3-Acetylaceto-BHB-Diglycerinester und andererseits ein aufgereinigtes, nach dem erfindungsgemäßen Verfahren erhaltenes Gemisch aus funktionalisiertem Mono-3-Acetylaceto-BHB-Diglycerinester, funktionalisiertem Di-3-Acetylaceto-BHB-Diglycerinester und funktionalisiertem Tri-3-Acetylaceto-BHB-Diglycerinester eingesetzt (Funktionalisierung mit Heptansäure oder Ölsäure oder Eicosapentaensäure/Docosahexaensäure-Gemischen).

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Die Versuche belegen, dass der 3-Acetylaceto-BHB-Diglycerinester sowie deren funktionalisierte Derivate jeweils einen geeigneten physiologischen Präkursor für die Ketokörper 3-Hydroxybuttersäure sowie Acetoacetat (und dadurch letztendlich wieder 3-Hydroxybuttersäure) zur Verwendung in den entsprechenden Ketokörpertherapien darstellen.

### Weitere Verdauversuche (Spaltungsversuche) von erfindungsgemäßen acetoacetylverkappten 3-Hydroxybuttersäure-PG(2)-Ester-Gemischen

### Spaltungsversuche mit Pankreatin

Jeweils 3,5 g eines wie zuvor beschrieben hergestellten Mono-3-Acetylaceto-BHB-Diglycerinester, Di-3-Acetylaceto-BHB-Diglycerinester, Tri-3-Acetylaceto-BHB-Diglycerinester und Tetra-3-Acetylaceto-BHB-Diglycerinester einerseits und ein aufgereinigtes, nach dem erfindungsgemäßen Verfahren erhaltenes Gemisch aus funktionalisiertem Mono-3-Acetylaceto-BHB-Diglycerinester, funktionalisiertem Di-3-Acetylaceto-BHB-Diglycerinester und funktionalisiertem Tri-3-Acetylaceto-BHB-Diglycerinester andererseits werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung des 3-Acetylaceto-BHB-Diglycerinester-Gemischs zu der freien 3-Hydroxybuttersäure und Acetoacetat, welches physiologisch wiederum zu 3-BHB bzw. zu 3-Hydroxybutyrat reduziert werden kann). Der Umsatz/Zeit-Verlauf der wässrigen Spaltung der erfindungsgemäßen Ester mittels Pankreatin, einschließlich Zunahme der Säurezahl über die Zeit, belegt die gewünschte Zersetzung des Edukts bzw. Eduktgemischs zu der freien Säure. Dies wird durch entsprechende Analytik bestätigt. Der Versuch belegt, dass sowohl der erfindungsgemäße 3-Acetylaceto-BHB-Diglycerinester als auch die funktionalisierten Derivate geeignete physiologische Präkursoren für 3-Hydroxybuttersäure für die entsprechenden Ketokörpertherapien darstellen. Die Versuche werden jeweils anhand der einzelnen Ester in Reinform wiederholt und verifiziert. Es werden vergleichbare Ergebnisse erhalten, d. h. sowohl die 3-Acetylaceto-BHB-Diglycerinester als auch die funktionalisierten Derivate werden jeweils durch Pankreatin gespalten.

Die zuvor geschilderten Spaltungsversuche belegen, dass die Polyglycerinester, der acetoacetylverkappten 3-Hydroxybuttersäure effiziente Präkursoren bzw. Metabolite der freien 3-Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

### Weitere Verdauversuche (Spaltungsversuche) von weiteren erfindungsgemäßen 3-Acetylaceto-BHB-Polyolester-Gemischen

Darüber hinaus werden auch die übrigen erfindungsgemäß hergestellten Polyolestergemische der 3-Acetylacetobuttersäure, wie zuvor beschrieben, in entsprechender Weise Verdauversuchen unterzogen und liefern analog Ergebnisse.

Auch die Spaltungsversuche belegen, dass die übrigen Polyolester der 3-Acetylacetobuttersäure effiziente Präkursoren bzw. Metabolite für die Ketokörper 3-Hydroxybuttersäure und Acetoacetat zur Verwendung in den entsprechenden Ketokörpertherapien darstellen, insbesondere im Hinblick auf ihre beabsichtigte Wirkung, welche zudem in physiologisch verträglicher bzw. physiologisch kompatibler Form vorliegen.

## Patentansprüche

1. Verfahren zur Herstellung von Polyglycerinestern von acetoacetylverkappter 3-Hydroxybuttersäure,
wobei mindestens eine Verbindung der allgemeinen Formel (I)
CH₃ - CH(OR²) - CH₂ - C(O)OR¹ (I)
wobei in der allgemeinen Formel (I) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt und der Rest R² einen Rest CH₃ - C(O) - CH₂ - C(O) - darstellt,
mit mindestens einem Polyglycerin der allgemeinen Formel (IIb)
HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 4, vorzugsweise 1 oder 2, besonders bevorzugt 1, darstellt,
umgesetzt wird,
wobei die Umsetzung in Abwesenheit von Lösemitteln durchgeführt wird und
wobei die Umsetzung in Gegenwart eines Enzyms als Katalysator durchgeführt wird, wobei der Katalysator nach der Umsetzung rezykliert wird,
so dass als Reaktionsprodukt mindestens ein acetoacetylverkappter 3-Hydroxybuttersäure-Polyglycerinester erhalten wird,
wobei bei der Umsetzung gleichzeitig die Verbindung gemäß der allgemeinen Formel (VI)
R¹ - OH (VI)
gebildet wird, wobei in der allgemeinen Formel (VI) der Rest R¹ Wasserstoff oder ein C₁-C₄-Alkyl, insbesondere ein C₁-C₄-Alkyl, vorzugsweise Methyl oder Ethyl, bevorzugt Ethyl, darstellt; wobei die Verbindung gemäß der allgemeinen Formel (VI) der Umsetzung kontinuierlich entzogen wird.

2. Verfahren nach einem Anspruch 1,
wobei die Verbindung der allgemeinen Formel (I), bezogen auf die Hydroxylgruppen des Polyglycerins (IIb) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei die Verbindung der allgemeinen Formel (I) und das Polyglycerin (IIb) in einem Molverhältnis von Verbindung der allgemeinen Formel (I) / Polyglycerin (IIb) in einem Bereich von 1 : 1 bis 10 : 1, insbesondere in einem Bereich von 2 : 1 bis 8 : 1, vorzugsweise in einem Bereich von 3 : 1 bis 6 : 1, eingesetzt werden.

3. Verfahren nach einem Anspruch 1 oder Anspruch 2,
wobei das Polyglycerin ein Diglycerin der Formel (IIc)
HO - CH₂ - CH(OH) - CH₂ - O - CH₂ - CH(OH) - CH₂ - OH (IIc)
ist.

4. Verfahren nach einem der vorangehenden Ansprüche,
wobei im Reaktionsprodukt nach erfolgter Umsetzung noch vorhandene Hydroxylgruppen zumindest teilweise, vorzugsweise vollständig, funktionalisiert, insbesondere verestert, werden.

5. Verfahren nach Anspruch 4,
wobei die Funktionalisierung, insbesondere Veresterung, der nach erfolgter Umsetzung noch vorhandenen Hydroxylgruppen im Reaktionsprodukt mit mindestens einem Carbonsäureanhydrid der allgemeinen Formel (VII)
R⁴ - O - R⁴ (VII)
wobei in der allgemeinen Formel (VII) der Rest R⁴ jeweils unabhängig voneinander, gleich oder verschieden, einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, durchgeführt wird.

6. Verfahren nach Anspruch 4,
wobei die Funktionalisierung, insbesondere Veresterung, der nach erfolgter Umsetzung noch vorhandenen Hydroxylgruppen im Reaktionsprodukt durch Reaktion mit mindestens einer Carbonsäure und/oder einem Carbonsäureester der allgemeinen Formel (IX)
R⁴ - O - R⁸ (IX)
wobei in der allgemeinen Formel (IX)
• der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt,
• der Rest R⁸ Wasserstoff oder ein C₁-C₄-Alkyl, insbesondere Methyl oder Ethyl, bevorzugt Wasserstoff, darstellt,
durchgeführt wird.

7. Verfahren nach Anspruch 6,
wobei die Carbonsäure und/oder der Carbonsäureester der allgemeinen Formel (IX) eine Fettsäure und/oder einen Fettsäureester, insbesondere eine C₅-C₃₄-Fettsäure und/oder einen C₅-C₃₄-Fettsäureester, vorzugsweise eine C₈-C₃₄-Fettsäure und/oder einen C₈-C₃₄-Fettsäureester, darstellt,
wobei die Fettsäure, vorzugsweise die C₅-C₃₄-Fettsäure, bevorzugt die C₈-C₃₄-Fettsäure, insbesondere in freier Form oder in Form von deren Ester oder Anhydrid, ausgewählt ist aus der Gruppe von Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Heneicosansäure, Behensäure, Lignocerinsäure, Cerotinsäure, Montansäure, Melissinsäure, Laccersäure, Geddinsäure, Undecylsäure, Myristoleinsäure, Palmitoleinsäure, Margaroleinsäure, Petroselinsäure, Ölsäure, Elaidinsäure, Vaccensäure, Gadoleinsäure, Cetoleinsäure, Erucasäure, Nervonsäure, Linolsäure, Linolensäuren, Calendulasäure, Punicinsäure, Eleostearinsäuren, Stearidonsäure, Arachidonsäure, Eicosapentaensäure, Docosadiensäure, Docosatetraensäure, Docosapentaensäure, Docosahexaensäure, und Tetracosahexaensäure sowie deren Mischungen.

8. Gegebenenfalls funktionalisierter acetoacetylverkappter 3-Hydroxybuttersäure-Polyglycerinester der allgemeinen Formel (IIIb")
R⁶O - CH₂ - CH(OR⁶) - CH₂ - [O - CH₂ - CH(OR⁶) - CH₂]ₚ - OR⁶ (IIIb")
wobei in der allgemeinen Formel (IIIb")
• die Variable p eine ganze Zahl 1 bis 4, vorzugsweise 1 oder 2, besonders bevorzugt 1, darstellt,
• der Rest R⁶ jeweils unabhängig voneinander, gleich oder verschieden, darstellt: Wasserstoff oder einen Rest CH₃ - CH(OR²) - CH₂ - C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

9. Gegebenenfalls funktionalisierter acetoacetylverkappter 3-Hydroxybuttersäure-Polyglycerinester nach Anspruch 8,
wobei der gegebenenfalls funktionalisierte acetoacetylverkappte 3-Hydroxybuttersäure-Polyglycerinester der allgemeinen Formel (IIIc")
R⁶O - CH₂ - CH(OR⁶) - CH₂ - O - CH₂ - CH(OR⁶) - CH₂ - OR⁶ (IIIc")
entspricht,
wobei in der allgemeinen Formel (IIIc") der Rest R⁶ jeweils unabhängig voneinander, gleich oder verschieden, darstellt: Wasserstoff oder einen Rest CH₃ - CH(OR²) - CH₂ -C(O) -, wobei der Rest R² einen Rest CH₃ - C(O) - CH₂ - C(O) - darstellt, oder einen Rest R⁴, wobei der Rest R⁴ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, vorzugsweise (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, einen Rest CH₃ - CH(OR²) - CH₂ - C(O) -, wie zuvor definiert, darstellt.

10. Gemisch, umfassend mindestens zwei, insbesondere mindestens drei, voneinander verschiedene gegebenenfalls funktionalisierte acetoacetylverkappte 3-Hydroxybuttersäure-Polyglycerinester, wie zuvor definiert.

11. Pharmazeutische Zusammensetzung, insbesondere Arzneimittel oder Medikament, umfassend einen oder mehrere gegebenenfalls funktionalisierte acetoacetylverkappte 3-Hydroxybuttersäure-Polyglycerinester gemäß einem der Ansprüche 8 bis 10.

12. Pharmazeutische Zusammensetzung nach Anspruch 11 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

13. Verwendung eines oder mehrerer gegebenenfalls funktionalisierter acetoacetylverkappter 3-Hydroxybuttersäure-Polyglycerinester gemäß einem der Ansprüche 8 bis 10 zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

14. Nahrungsmittel- und/oder Lebensmittelerzeugnis, umfassend einen oder mehrere gegebenenfalls funktionalisierte acetoacetylverkappte 3-Hydroxybuttersäure-Polyglycerinester gemäß einem der Ansprüche 8 bis 10.

15. Verwendung eines oder mehrerer gegebenenfalls funktionalisierter acetoacetylverkappter 3-Hydroxybuttersäure-Polyglycerinester gemäß einem der Ansprüche 8 bis 10 in einem Nahrungsmittel- und/oder Lebensmittelerzeugnis.

## Claims

1. A method for the preparation of polyglycerol esters of acetoacetyl-capped 3-hydroxybutyric acid,
wherein at least one compound of the general formula (I)
CH₃ - CH(OR²) - CH₂ - C(O)OR¹ (I)
wherein, in general formula (I), the radical R¹ represents hydrogen or a C₁-C₄-alkyl, in particular a C₁-C₄-alkyl, preferably methyl or ethyl, more preferably ethyl, and the radical R² represents a radical CH₃ - C(O) - CH₂ - C(O) -,
is reacted with at least one polyglycerol of the general formula (IIb)
HO - CH₂ - CH(OH) - CH₂ - [O - CH₂ - CH(OH) - CH₂]ₚ - OH (IIb)
wherein, in the general formula (IIb), the variable p represents an integer from 1 to 4, preferably 1 or 2, most preferably 1,
wherein the reaction is carried out in the absence of solvents, and
wherein the reaction is carried out in the presence of an enzyme as a catalyst, wherein the catalyst is recycled after the reaction,
so that at least one acetoacetyl-capped 3-hydroxybutyric acid polyglycerol ester is obtained as the reaction product,
wherein, during the reaction, at the same time the compound according to the general formula (VI)
R¹ - OH (VI)
is formed, wherein, in general formula (VI), the radical R¹ represents hydrogen or a C₁-C₄-alkyl, in particular a C₁-C₄-alkyl, preferably methyl or ethyl, more preferably ethyl; wherein the compound according to general formula (VI) is continuously removed from the reaction.

2. The method according to claim 1,
wherein the compound of general formula (I), based on the hydroxyl groups of the polyglycerol (IIb), is used in molar amounts in a range from an equimolar amount up to a molar excess of 200 mol%, in particular in a range from an equimolar amount up to a molar excess of 150 mol%, preferably in a range from an equimolar amount up to a molar excess of 100 mol%; and/or
wherein the compound of general formula (I) and the polyglycerol (Ilb) are used in a molar ratio of compound of general formula (I) / polyglycerol (IIb) in a range from 1 : 1 to 10 : 1, in particular in a range from 2 : 1 to 8 : 1, preferably in a range from 3 : 1 to 6 : 1.

3. The method according to claim 1 or claim 2,
wherein the polyglycerol is a diglycerol of formula (IIc)
HO - CH₂ - CH(OH) - CH₂ - O - CH₂ - CH(OH) - CH₂ - OH (IIc).

4. The method according to any of the preceding claims,
wherein hydroxyl groups still present in the reaction product after the reaction has taken place are at least partially, preferably completely, functionalized, in particular esterified.

5. The method according to claim 4,
wherein the functionalization, in particular esterification, of the hydroxyl groups still present in the reaction product after the reaction has taken place is carried out with at least one carboxylic acid anhydride of the general formula (VII)
R⁴ - O - R⁴ (VII)
wherein, in general formula (VII), the radical R⁴ in each case independently of one another, being the same or different, represents a radical of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -.

6. The method according to claim 4,
wherein the functionalization, in particular esterification, of the hydroxyl groups still present in the reaction product after the reaction has taken place is carried out by reaction with at least one carboxylic acid and/or a carboxylic acid ester of the general formula (IX)
R⁴ - O - R⁸ (IX)
wherein, in general formula (IX),
• the radical R⁴ represents a radical of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -,
• the radical R⁸ represents hydrogen or a C₁-C₄-alkyl, in particular methyl or ethyl, preferably hydrogen.

7. The method according to claim 6,
wherein the carboxylic acid and/or the carboxylic acid ester of general formula (IX) represents a fatty acid and/or a fatty acid ester, in particular a C₅-C₃₄ fatty acid and/or a C₅-C₃₄ fatty acid ester, preferably a C₈-C₃₄ fatty acid and/or a C₈-C₃₄ fatty acid ester,
wherein the fatty acid, preferably the C₅-C₃₄ fatty acid, more preferably the C₈-C₃₄ fatty acid, in particular in free form or in the form of its ester or anhydride, is selected from the group consisting of caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachidic acid, heneicosanoic acid, behenic acid, lignoceric acid, cerotic acid, montanic acid, melissic acid, lacceroic acid, geddic acid, undecylic acid, myristoleic acid, palmitoleic acid, margaroleic acid, petroselinic acid, oleic acid, elaidic acid, vaccenic acid, gadoleic acid, cetoleic acid, erucic acid, nervonic acid, linoleic acid, linolenic acids, calendulic acid, punicic acid, eleostearic acids, stearidonic acid, arachidonic acid, eicosapentaenoic acid, docosadienoic acid, docosatetraenoic acid, docosapentaenoic acid, docosahexaenoic acid, and tetracosahexaenoic acid, as well as mixtures thereof.

8. An optionally functionalized acetoacetyl-capped 3-hydroxybutyric acid polyglycerol ester of the general formula (IIIb")
R⁶O - CH₂ - CH(OR⁶) - CH₂ - [O - CH₂ - CH(OR⁶) - CH₂]ₚ - OR⁶ (IIIb")
wherein, in the general formula (IIIb"),
• the variable p represents an integer from 1 to 4, preferably 1 or 2, more preferably 1,
• the radical R⁶ in each case independently of one another, being the same or different, represents: hydrogen or a radical CH₃ - CH(OR²) - CH₂ - C(O) -, wherein the radical R² represents a radical CH₃ - C(O) - CH₂ - C(O) -, or a radical R⁴, wherein the radical R⁴ represents a radical of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -, but with the proviso that at least one radical R⁶, in particular at least two radicals R⁶, does not represent hydrogen, and with the proviso that at least one radical R⁶, in particular at least two radicals R⁶, represents a radical CH₃ - CH(OR²) - CH₂ - C(O) -, as defined above.

9. The optionally functionalized acetoacetyl-capped 3-hydroxybutyric acid polyglycerol ester according to claim 8,
wherein the optionally functionalized acetoacetyl-capped 3-hydroxybutyric acid polyglycerol ester corresponds to the general formula (IIIc")
R⁶O - CH₂ - CH(OR⁶) - CH₂ - O - CH₂ - CH(OR⁶) - CH₂ - OR⁶ (IIIc"),
wherein, in the general formula (IIIc"), the radical R⁶ in each instance independently of one another, being the same or different, represents: hydrogen or a radical CH₃ - CH(OR²) - CH₂ - C(O) -, wherein the radical R² represents a radical CH₃ - C(O) - CH₂ - C(O) -, or a radical R⁴, wherein the radical R⁴ represents a radical of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -, but with the proviso that at least one radical R⁶, in particular at least two radicals R⁶, does not represent hydrogen, and with the proviso that at least one radical R⁶, in particular at least two radicals R⁶, represents a radical CH₃ - CH(OR²) - CH₂ - C(O) -, as defined above.

10. A mixture comprising at least two, in particular at least three, mutually different, optionally functionalized acetoacetyl-capped 3-hydroxybutyric acid polyglycerol esters, as defined above.

11. A pharmaceutical composition, in particular a drug or medicament, comprising one or more optionally functionalized acetoacetyl-capped 3-hydroxybutyric acid polyglycerol esters according to any of claims 8 to 10.

12. The pharmaceutical composition according to claim 11 for use in the prophylactic and/or therapeutic treatment of diseases of the human or animal body, in particular diseases associated with a disorder of energy metabolism, in particular keto body metabolism, such as, in particular, traumatic brain injury, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis, and amyotrophic lateral sclerosis, lipid metabolism diseases such as glucose transporter deficiency (GLUT1-defect), VL-FAOD, and mitochondriapathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, particularly ulcerative colitis and Crohn's disease, lysosomal storage diseases such as sphingolipidosis, particularly Niemann-Pick disease, diabetes mellitus, and effects or side effects of chemotherapy.

13. Use of one or more optionally functionalized acetoacetyl-capped 3-hydroxybutyric acid polyglycerol esters according to any of claims 8 to 10 for the preparation of a pharmaceutical composition for the prophylactic and/or therapeutic treatment of diseases of the human or animal body, in particular diseases associated with a disorder of energy metabolism, in particular keto body metabolism, such as, in particular, traumatic brain injury, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis, and amyotrophic lateral sclerosis, lipid metabolism diseases such as glucose transporter deficiency (GLUT1-defect), VL-FAOD, and mitochondriapathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, particularly ulcerative colitis and Crohn's disease, lysosomal storage diseases such as sphingolipidosis, particularly Niemann-Pick disease, diabetes mellitus, and effects or side effects of chemotherapy.

14. A food and/or nutritional product comprising one or more optionally functionalized acetoacetyl-capped 3-hydroxybutyric acid polyglycerol esters according to any of claims 8 to 10.

15. Use of one or more optionally functionalized acetoacetyl-capped 3-hydroxybutyric acid polyglycerol esters according to any of claims 8 to 10 in a food and/or nutritional product.

## Revendications

1. Procédé de préparation d'esters de polyglycérol d'acide 3-hydroxybutyrique coiffé par acétoacétyle,
où au moins un composé de formule générale (I)
CH₃ - CH(OR²) - CH₂ - C(O)OR¹ (I)
où, dans la formule générale (I), le radical R¹ représente hydrogène ou C₁-C₄ alkyle, en particulier C₁-C₄ alkyle, de préférence méthyle ou éthyle, de préférence éthyle, et le radical R² représente un radical CH₃ - C(O) - CH₂ - C(O) -,
est mis en réaction avec au moins un polyglycérol de formule générale (Ilb)
HO - CH₂ - CH(OH) - CH₂ - [O - CH₂ - CH(OH) - CH₂]ₚ - OH (IIb)
où, dans la formule générale (IIb), la variable p représente un nombre entier de 1 à 4, de préférence 1 ou 2, de façon particulièrement préférée 1,
où la réaction est réalisée en l'absence de solvants et
où la réaction est réalisée en présence d'une enzyme comme catalyseur, où le catalyseur est recyclé après la réaction,
de sorte qu'au moins un ester de polyglycérol d'acide 3-hydroxybutyrique coiffé par acétoacétyle est obtenu comme produit de réaction,
où, lors de la réaction, le composé selon la formule générale (VI)
R¹ - OH (VI)
est formé, où, dans la formule générale (VI), le radical R¹ représente hydrogène ou C₁-C₄ alkyle, en particulier C₁-C₄ alkyle, de préférence méthyle ou éthyle, de préférence éthyle; où le composé selon la formule générale (VI) est retiré de la réaction en continu.

2. Procédé selon la revendication 1,
où le composé de formule générale (I), par rapport aux groupes hydroxyle du polyglycérol (Ilb), est utilisé en quantités molaires dans un intervalle de quantité équimolaire jusqu'à un excès molaire de 200 % en moles, en particulier dans un intervalle de quantité équimolaire jusqu'à un excès molaire de 150 % en moles, de préférence dans un intervalle de quantité équimolaire jusqu'à un excès molaire de 100 % en moles; et/ou
où le composé de formule générale (I) et le polyglycérol (IIb) sont utilisés dans un rapport molaire composé de formule générale (I)/polyglycérol (IIb) dans un intervalle de 1 : 1 à 10 : 1, en particulier dans un intervalle de 2 : 1 à 8 : 1, de préférence dans un intervalle de 3 : 1 à 6 : 1.

3. Procédé selon la revendication 1 ou la revendication 2,
où le polyglycérol est un diglycérol de formule (Ilc)
HO - CH₂ - CH(OH) - CH₂ - O - CH₂ - CH(OH) - CH₂ - OH (IIc).

4. Procédé selon l'une quelconque des revendications précédentes,
où des groupes hydroxyle encore présents dans le produit de réaction après la réaction sont fonctionnalisés, en particulier estérifiés, au moins partiellement, de préférence complètement.

5. Procédé selon la revendication 4,
où la fonctionnalisation, en particulier l'estérification, des groupes hydroxyle encore présents dans le produit de réaction après la réaction est réalisée avec au moins un anhydride d'acide carboxylique de formule générale (VII)
R⁴ - O - R⁴ (VII)
où, dans la formule générale (VII), le radical R⁴, chacun indépendamment, de façon identique ou différente, représente un radical du type (C₁-C₃₃ alkyle) - C(O) -, en particulier (C₄-C₃₃ alkyle) - C(O) -, de préférence (C₇-C₃₃ alkyle) - C(O) -, linéaire (à chaîne droite) ou ramifié, saturé ou mono- ou polyinsaturé.

6. Procédé selon la revendication 4,
où la fonctionnalisation, en particulier l'estérification, des groupes hydroxyle encore présents dans le produit de réaction après la réaction est réalisée par réaction avec au moins un acide carboxylique et/ou un ester d'acide carboxylique de formule générale (IX)
R⁴ - O - R⁸ (IX)
où, dans la formule générale (IX),
• le radical R⁴ représente un radical du type (C₁-C₃₃ alkyle) - C(O) -, en particulier (C₄-C₃₃ alkyle) - C(O) -, de préférence (C₇-C₃₃ alkyle) - C(O) -, linéaire (à chaîne droite) ou ramifié, saturé ou mono- ou polyinsaturé,
• le radical R⁸ représente hydrogène ou C₁-C₄ alkyle, en particulier méthyle ou éthyle, de préférence hydrogène.

7. Procédé selon la revendication 6,
où l'acide carboxylique et/ou l'ester d'acide carboxylique de formule générale (IX) représente un acide gras et/ou un ester d'acide gras, en particulier un acide gras en C₅-C₃₄ et/ou un ester d'acide gras en C₅-C₃₄, de préférence un acide gras en C₈-C₃₄ et/ou un ester d'acide gras en C₈-C₃₄,
où l'acide gras, de préférence l'acide gras en C₅-C₃₄, de préférence l'acide gras en C₈-C₃₄, en particulier sous forme libre ou sous forme d'un ester ou d'un anhydride de celuici, est choisi dans le groupe constitué par l'acide caprylique, l'acide pélargonique, l'acide caprique, l'acide undécanoïque, l'acide laurique, l'acide tridécanoïque, l'acide myristique, l'acide pentadécanoïque, l'acide palmitique, l'acide margarique, l'acide stéarique, l'acide nonadécanoïque, l'acide arachidique, l'acide hénéicosanoïque, l'acide béhénique, l'acide lignocérique, l'acide cérotique, l'acide montanique, l'acide mélissinique, l'acide laccérique, l'acide géddinique, l'acide undécylique, l'acide myristoléique, l'acide palmitoléique, l'acide margaroléique, l'acide pétrosélinique, l'acide oléique, l'acide élaïdique, l'acide vaccénique, l'acide gadoléique, l'acide cétooléique, l'acide érucique, l'acide nervonique, l'acide linoléique, l'acide linolénique, l'acide caldulaïque, l'acide punicique, l'acide éléostéarique, l'acide stéaridonique, l'acide arachidonique, l'acide eicosapentaénoïque, l'acide docosadiénoïque, l'acide docosatétraénoïque, l'acide docosapentaénoïque, l'acide docosahexaénoïque et l'acide tétracosahexaénoïque, ainsi que leurs mélanges.

8. Ester de polyglycérol d'acide 3-hydroxybutyrique coiffé par acétoacétyle éventuellement fonctionnalisé de formule générale (IIIb")
R⁶O - CH₂ - CH(OR⁶) - CH₂ - [O - CH₂ - CH(OR⁶) - CH₂]ₚ - OR⁶ (IIIb")
où, dans la formule générale (IIIb"),
• la variable p représente un nombre entier de 1 à 4, de préférence 1 ou 2, de façon particulièrement préférée 1,
• le radical R⁶, chacun indépendamment, de façon identique ou différente, représente hydrogène ou un radical CH₃ - CH(OR²) - CH₂ - C(O) -, où le radical R² représente un radical CH₃ - C(O) - CH₂ - C(O) -, ou un radical R⁴, où le radical R⁴ représente un radical du type (C₁-C₃₃ alkyle) - C(O) -, en particulier (C₄-C₃₃ alkyle) - C(O) -, de préférence (C₇-C₃₃ alkyle) - C(O) -, linéaire (à chaîne droite) ou ramifié, saturé ou mono- ou polyinsaturé, mais à condition qu'au moins un radical R⁶, en particulier au moins deux radicaux R⁶, ne représente pas hydrogène, et à condition qu'au moins un radical R⁶, en particulier au moins deux radicaux R⁶, représente un radical CH₃-CH(OR²) - CH₂ - C(O) -, comme défini précédemment.

9. Ester de polyglycérol d'acide 3-hydroxybutyrique coiffé par acétoacétyle éventuellement fonctionnalisé selon la revendication 8,
où l'ester de polyglycérol d'acide 3-hydroxybutyrique coiffé par acétoacétyle éventuellement fonctionnalisé corresponds à la formule générale (IIIc")
R⁶O - CH₂ - CH(OR⁶) - CH₂ - O - CH₂ - CH(OR⁶) - CH₂ - OR⁶ (IIIc")
où, dans la formule générale (IIIc"), le radical R⁶, chacun indépendamment, de façon identique ou différente, représente hydrogène ou un radical CH₃ - CH(OR²) - CH₂ - C(O) -, où le radical R² représente un radical CH₃ - C(O) - CH₂ - C(O) -, ou un radical R⁴, où le radical R⁴ représente un radical du type (C₁-C₃₃ alkyle) - C(O) -, en particulier (C₄-C₃₃ alkyle) - C(O) -, de préférence (C₇-C₃₃ alkyle) - C(O) -, linéaire (à chaîne droite) ou ramifié, saturé ou mono- ou polyinsaturé, mais à condition qu'au moins un radical R⁶, en particulier au moins deux radicaux R⁶, ne représente pas hydrogène, et à condition qu'au moins un radical R⁶, en particulier au moins deux radicaux R⁶, représente un radical CH₃ - CH(OR²) - CH₂ - C(O) -, comme défini précédemment.

10. Mélange comprenant au moins deux, en particulier au moins trois, esters de polyglycérol d'acide 3-hydroxybutyrique coiffé par acétoacétyle éventuellement fonctionnalisés, différents les uns des autres, comme défini précédemment.

11. Composition pharmaceutique, en particulier médicament ou drogue, comprenant un ou plusieurs esters de polyglycérol d'acide 3-hydroxybutyrique coiffé par acétoacétyle éventuellement fonctionnalisés selon l'une quelconque des revendications 8 à 10.

12. Composition pharmaceutique selon la revendication 11 pour utilisation dans le traitement prophylactique et/ou thérapeutique de maladies du corps humain ou animal, en particulier de maladies liées à un trouble du métabolisme énergétique, en particulier du métabolisme des corps cétoniques, comme en particulier un traumatisme crânien, un accident vasculaire cérébral, l'hypoxie, les maladies cardiovasculaires comme l'infarctus du myocarde, le syndrome de renutrition inappropriée, l'anorexie, l'épilepsie, les maladies neurodégénératives comme la démence, la maladie d'Alzheimer, la maladie de Parkinson, la sclérose en plaques et la sclérose latérale amyotrophique, les maladies du métabolisme des lipides comme le déficit en transporteur de glucose (GLUT1 déficit), le VL-FAOD et les mitochondriopathies comme le déficit en thiolase mitochondriale, la maladie de Huntington, les cancers comme les lymphomes à cellules T, les astrocytomes et les glioblastomes, le VIH, les maladies rhumatoïdes comme la polyarthrite rhumatoïde et l'arthrite rhumatoïde, les maladies du tractus gastro-intestinal comme les maladies intestinales inflammatoires chroniques, en particulier la rectocolite hémorragique et la maladie de Crohn, les maladies lysosomales comme les sphingolipidoses, en particulier la maladie de Niemann-Pick, le diabète sucré et les effets ou effets secondaires de chimiothérapies.

13. Utilisation d'un ou plusieurs esters de polyglycérol d'acide 3-hydroxybutyrique coiffé par acétoacétyle éventuellement fonctionnalisés selon l'une quelconque des revendications 8 à 10 pour la préparation d'un médicament destiné au traitement prophylactique et/ou thérapeutique de maladies du corps humain ou animal, en particulier de maladies liées à un trouble du métabolisme énergétique, en particulier du métabolisme des corps cétoniques, comme en particulier un traumatisme crânien, un accident vasculaire cérébral, l'hypoxie, les maladies cardiovasculaires comme l'infarctus du myocarde, le syndrome de renutrition inappropriée, l'anorexie, l'épilepsie, les maladies neurodégénératives comme la démence, la maladie d'Alzheimer, la maladie de Parkinson, la sclérose en plaques et la sclérose latérale amyotrophique, les maladies du métabolisme des lipides comme le déficit en transporteur de glucose (GLUT1 déficit), le VL-FAOD et les mitochondriopathies comme le déficit en thiolase mitochondriale, la maladie de Huntington, les cancers comme les lymphomes à cellules T, les astrocytomes et les glioblastomes, le VIH, les maladies rhumatoïdes comme la polyarthrite rhumatoïde et l'arthrite rhumatoïde, les maladies du tractus gastro-intestinal comme les maladies intestinales inflammatoires chroniques, en particulier la rectocolite hémorragique et la maladie de Crohn, les maladies lysosomales comme les sphingolipidoses, en particulier la maladie de Niemann-Pick, le diabète sucré et les effets ou effets secondaires de chimiothérapies.

14. Produit alimentaire et/ou vivrier, comprenant un ou plusieurs esters de polyglycérol d'acide 3-hydroxybutyrique coiffé par acétoacétyle éventuellement fonctionnalisés selon l'une quelconque des revendications 8 à 10.

15. Utilisation d'un ou plusieurs esters de polyglycérol d'acide 3-hydroxybutyrique coiffé par acétoacétyle éventuellement fonctionnalisés selon l'une quelconque des revendications 8 à 10 dans un produit alimentaire et/ou vivrier.
